# EUROPEAN PATENT SPECIFICATION

(11) **EP 1 062 240 B1**
(45) Date of publication and mention of the grant of the patent: **28.04.2010**
(21) Application number: 99906078.3
(22) Date of filing: 25.02.1999
(51) Int. Cl.: C07K 14/605, A61K 38/26

(54) **N-TERMINALLY MODIFIED GLP-1 DERIVATIVES**
N-TERMINAL VERÄNDERTE GLP-1 ABKÖMMLINGE
DERIVES DE GLP-1 MODIFIES A L'EXTREMITE N-TERMINALE

(30) Priority: 27.02.1998 DK 26898; 27.02.1998 DK 26398; 08.04.1998 DK 50898
(43) Date of publication of application: 27.12.2000
(73) Proprietor: NOVO NORDISK A/S, 2880 Bagsværd (DK)
(72) Inventor: KNUDSEN, Liselotte, Bjerre, DK-2500 Valby (DK); HUUSFELDT, Per, Olaf, DK-2100 København Ø (DK); NIELSEN, Per, Franklin, DK-3500 Værløse (DK); MADSEN, Kjeld, DK-3500 Værløse (DK)
(74) Representative: Kjerrumgaard, Lars Bo
(86) International application number: PCT/DK1999/000085
(87) International publication number: WO 1999/043707

(56) References cited:
- EP-A2- 0 708 179
- WO-A1-94/26778
- WO-A1-95/31214
- WO-A1-96/29342
- WO-A1-97/31943
- WO-A1-98/08871
- US-A- 5 545 618
- US-A- 5 614 492
- US-A- 5 631 224

## Description

### FIELD OF THE INVENTION

The present invention relates to novel derivatives of human glucagon-like peptide-1 (GLP-1) and fragments and analogues thereof having a protracted profile of action and to the use of such derivatives in pharmaceutical compositions.

### BACKGROUND OF THE INVENTION

GLP-1 (Glucacon-Like-Peptide-1) is an important gut hormone with regulatory function in glucose metabolism and gastrointestinal secretion and metabolism. Human GLP-1 is a 37 amino acid residue peptide originating from preproglucagon which is synthesised *i.a*. in the L-cells in the distal ileum, in the pancreas and in the brain. Processing of preproglucagon to give GLP-1 (7-36)amide, GLP-1 (7-37) and GLP-2 occurs mainly in the L-cells.

WO 87/06941 (The General Hospital Corporation) disclose peptide fragments which comprises GLP-1 (7-37) and functional derivatives thereof and to its use as an insulinotropic agent.

WO 90/11296 (The General Hospital Corporation) disclose peptide fragments which comprise GLP-1 (7-36) and functional derivatives thereof and have an insulinotropic activity which exceeds the insulinotropic activity of GLP-1 (1-36) or GLP-1 (1-37) and to their use as insulinotropic agents.

The amino acid sequence of GLP-1 (7-36)amide and GLP-1 (7-37) is: 
7 8 9 10 11 12 13 14 15 16 17 18 19 20 21 22 23
His-Ala-Glu-Gly-Thr-Phe-Thr-Ser-Asp-Val-Ser-Ser-Tyr-Leu-Glu-Gly-Gln-

24 25 26 27 28 29 30 31 32 33 34 35 36 (I)
Ala-Ala-Lys-Glu-Phe-Ile-Ala-Trp-Leu-Val-Lys-Gly-Arg-X
 wherein X is NH₂ for GLP-1 (7-36)amide and X is Gly-OH for GLP-1 (7-37).

WO 91/11457 (Buckley et al.) discloses analogues of the active GLP-1 peptides 7-34, 7-35, 7-36, and 7-37.

WO 98/08871 discloses GLP-1 derivatives in which a lipophilic substituent is attached to at least one amino acid residue. The lipophilic substituents are in particular long-chain groups containing e.g. 12-24 carbon atoms.

EP 0708179-A2 (Eli Lilly & Co.) discloses GLP-1 analogues and derivatives that include an N-terminal imidazole group and optionally an unbranched C₆-C₁₀ acyl group in attached to the lysine residue in position 34.

It is an object of the present invention to provide improved GLP-1 derivatives.

### SUMMARY OF THE INVENTION

The present invention relates to a GLP-1 derivative having a protracted profile of action relative to GLP-1 (7-37) and of the formula III: 
7 8 9 10 11 12 13 14 15 16 17
Xaa-Xaa-Xaa-Gly-Xaa-Phe-Thr-Xaa-Asp-Xaa-Xaa-

18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa (III)
 wherein
Xaa at position 7
is selected from the group consisting of: 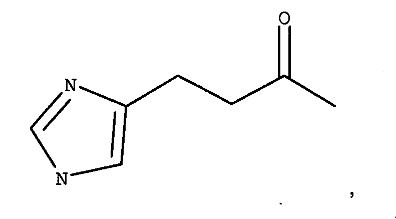 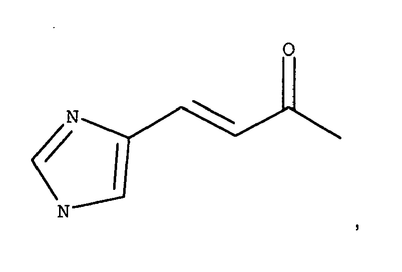 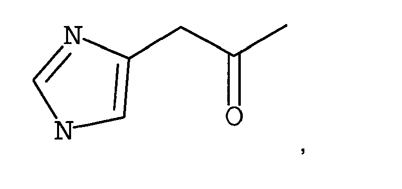 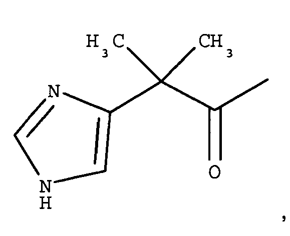 and 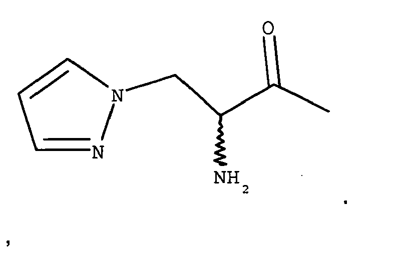 Xaa at position 8 is Ala, Gly, Ser, Thr, or Val,
Xaa at position 9 is Glu,
Xaa at position 11 is Thr,
Xaa at position 14 is Ser,
Xaa at position 16 is Val,
Xaa at position 17 is Ser,
Xaa at position 18 is Ser, Glu, Asp, or Lys,
Xaa at position 19 is Tyr, Glu, Asp, or Lys,
Xaa at position 20 is Leu, Glu, Asp, or Lys,
Xaa at position 21 is Glu, Asp, or Lys,
Xaa at position 22 is Gly, Glu, Asp, or Lys,
Xaa at position 23 is Gin, Glu, Asp, or Lys,
Xaa at position 24 is Ala, Glu, Asp, or Lys,
Xaa at position 25 is Ala, Glu, Asp, or Lys,
Xaa at position 26 is Lys, Arg, Glu, or Asp,
Xaa at position 27 is Glu, Asp, or Lys,
Xaa at position 30 is Ala, Glu, Asp, or Lys,
Xaa at position 31 is Trp, Glu, Asp, or Lys,
Xaa at position 32 is Leu, Glu, Asp, or Lys,
Xaa at position 33 is Val, Glu, Asp, or Lys,
Xaa at position 34 is Lys, Arg, Glu, or Asp,
Xaa at position 35 is Gly, Glu, Asp, or Lys,
Xaa at position 36 is Arg, Lys, Glu, or Asp,
Xaa at position 37 is Gly, Glu, Asp, or Lys,
Xaa at position 38 is deleted,
Xaa at position 39 is deleted,
Xaa at position 40 is deleted,
Xaa at position 41 is deleted,
Xaa at position 42 is deleted,
Xaa at position 43 is deleted,
Xaa at position 44 is deleted, and
Xaa at position 45 is deleted, or
(a) a C-1-6-ester thereof, (b) an amide, C-1-6-alkylamide, or C-1-6-dialkylamide thereof, and/or (c) a pharmaceutically acceptable salt thereof,
wherein
(i) a lipophilic substituent comprising from 12 to 24 carbon atoms is attached via a spacer to one or more of (a) the amino group of the N-terminal amino acid, (b) the carboxy group of the C-terminal amino acid, (c) the ε-amino group of Lys, and/or (d) the carboxy group which is part of the R group of Asp or Glu, and
(ii) the total number of different amino acids of the GLP-1 peptide as compared to the corresponding native form of GLP-1 is one, two, three, four, five, or six.

In its broadest aspect, the present invention discloses derivatives of GLP-1(7-B) and analogues thereof. The derivatives according to the invention have interesting pharmacological properties, including a protracted profile of action. The derivatives also are more metabolically and physically stable, and more soluble.

The GLP-1 derivatives and analogues of the present invention comprise a lipophilic substituent comprising from12 to 24 carbon atoms (via a spacer) attached to at least one amino acid residue and the N-terminal amino acid, i.e., the histidine residue at position 7 is modified. The lipophilic substituent is in particular a long-chain group of the type described in WO 98/08871 (Novo Nordisk A/S), and the N-terminal substituent comprises an optionally substituted 5- or 6-membered ring system, viz. an imidazole.

GLP-1 derivatives comprising a parent peptide of formula II are also generally disclosed herein, in which formula II is

A-HN-GLP-1(8-B)-X (II)

wherein
A is: 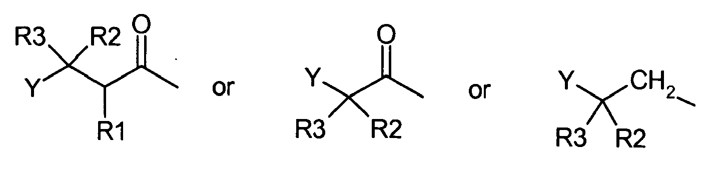 wherein R¹, R² and R³ are independently H, lower alkyl having 1 to 6 carbon atoms, optionally substituted phenyl, NH₂, NH-CO-(lower alkyl), -OH, lower alkoxy having 1 to 6 carbon atoms, halogen, SO₂-(lower alkyl) or CF₃, said phenyl is optionally substituted with at least one group selected from NH₂, -OH, lower alkyl or lower alkoxy having 1-6 carbon atoms, halogen, SO₂-(lower alkyl), NH-CO-(lower alkyl) or CF₃, or R¹ and R² may together form a bond;
Y is a five or six membered ring system selected from the group consisting of: 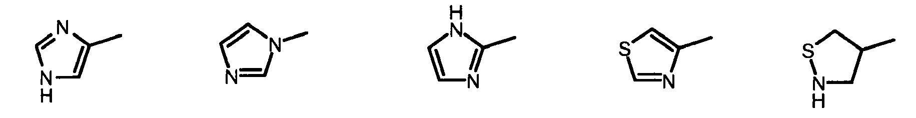 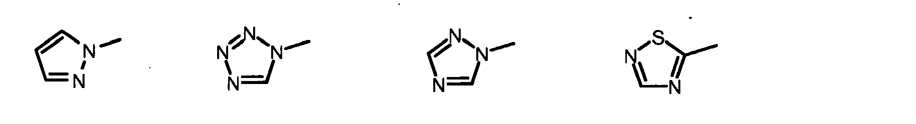 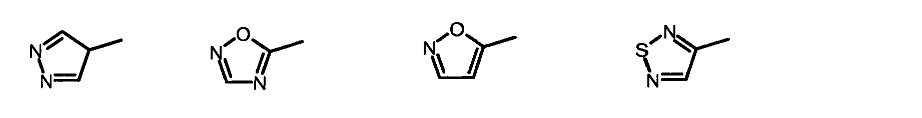 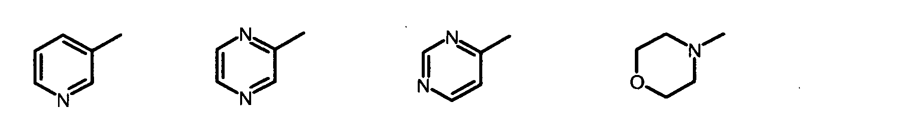 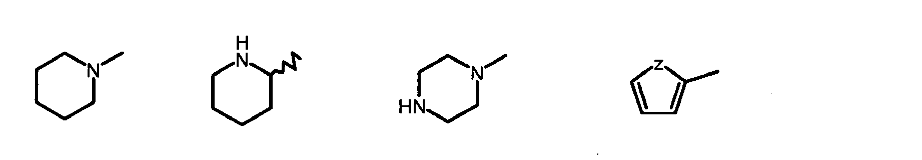 wherein Z is N, O or S, said ring system is optionally substituted with one or more functional groups selected from the group consisting of NH₂, NO₂, OH, lower alkyl, lower alkoxy, halogen, CF₃ and aryl;
B is an integer in the range of 35-45; and
X is-OH, -NH₂, or a C₁₋₆ alkyl amide or C₁₋₆ dialkyl amide group;
or an analogue thereof;
said GLP-1 derivative or analogue comprising a lipophilic substituent attached to at least one amino acid residue thereof.

In particular, the invention also discloses GLP-1 derivatives of formula II

A-HN-GLP-1(8-B)-X (II)

wherein
A is: 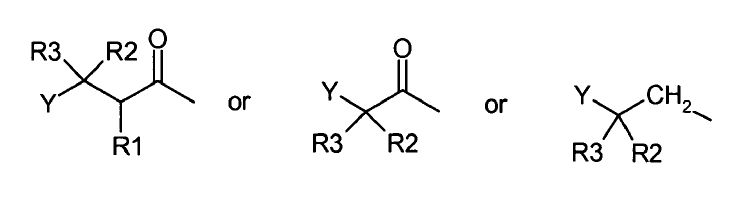 wherein R¹, R² and R³ are independently H, lower alkyl, optionally substituted phenyl, NH₂, NH-CO-(lower alkyl), -OH, lower alkoxy, halogen, SO₂-(lower alkyl) or CF₃, wherein said phenyl is optionally substituted with at least one group selected from NH₂, -OH, lower alkyl or lower alkoxy having 1-6 carbon atoms, halogen, SO₂-(lower alkyl), NH-CO-(lower alkyl) or CF₃, or R¹ and R² may together form a bond; and Y is a five or six membered ring system selected from the group consisting of: 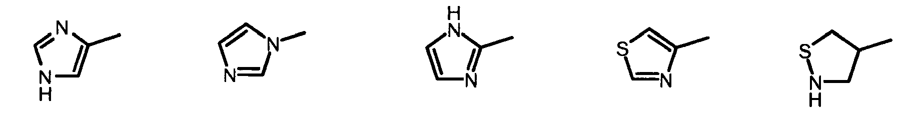 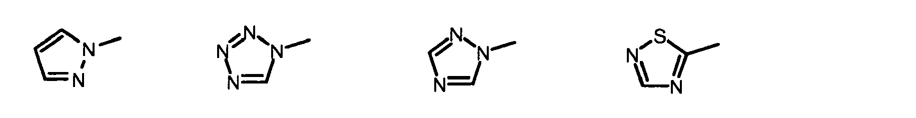 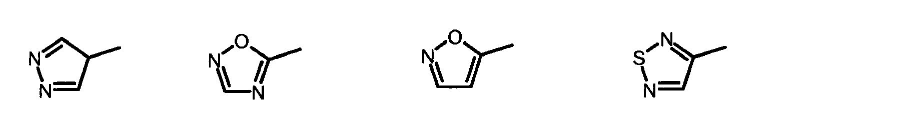 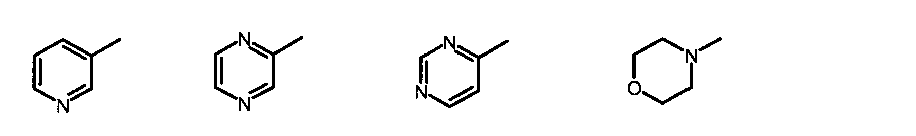 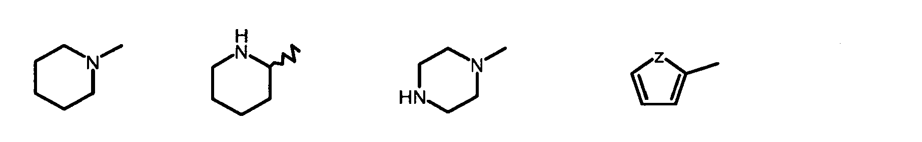 wherein Z is N, O or S, and said ring system is optionally substituted with one or more functional groups selected from the group consisting of NH₂, NO₂, OH, lower alkyl, lower alkoxy, halogen, CF₃ and aryl (i.e., optionally substituted phenyl, as define above), provided that A is not histidine;
B is an integer in the range of 35-45; and
X is -OH, -NH₂, or a C₁₋₆ alkyl amide or C₁₋₆ dialkyl amide group;
or an analogue thereof;
wherein a lipophilic substituent (optionally via a spacer) is attached to at least one amino acid residue provided that when the lipophilic substituent is an acyl group and no spacer is present, then the acyl group contains at least 12 carbon atoms.

### DETAILED DESCRIPTION OF THE INVENTION

A simple system is used to describe the GLP-1 derivatives of the present invention. For example, Gly⁸-GLP-1(7-37) designates a peptide which relates to GLP-1 (1-37) by the deletion of the amino acid residues at positions 1 to 6 and the substitution of the naturally occurring amino acid residue in position 8 (Ala) with Gly. Similarly, Lys³⁴(N^{ε}-tetradecanoyl)-GLP-1 (7-37) designates GLP-1 (7-37) wherein the ε-amino group of the Lys residue in position 34 has been tetradecanoylated. Where a reference is made to C-terminally extended GLP-1 analogues, the amino acid residue in position 38 is Arg unless otherwise indicated, the amino acid residue in position 39 is also Arg unless otherwise indicated and the amino acid residue in position 40 is Asp unless otherwise indicated. Also, if a C-terminally extended analogue extends to position 41, 42, 43, 44 or 45, the amino acid sequence of this extension is as in the corresponding sequence in human preproglucagon unless otherwise indicated.

### GLP-1 Analogs

The present invention also relates to derivatives of analogs of GLP-1. The term "analogue" is defined herein as a peptide which relates to a parent peptide by the substitution of one or more amino acid residues of the parent peptide with other amino acid residue(s).

In a further preferred embodiment, a parent peptide for a derivative of the invention is
Arg²⁶-GLP-1 (7-37); Arg³⁴-GLP-1(7-37); Lys³⁶-GLP-1(7-37);
Arg^{26,34}Lys³⁶-GLP-1 (7-37);
Arg²⁶Lys³⁶-GLP-1 (7-37); Arg³⁴Lys³⁶-GLP-1 (7-37);
Gly⁸Arg²⁶-GLP-1(7-37); Gly⁸Arg³⁴-GLP-1(7-37);
Gly⁸Lys³⁶-GLP-1(7-37); Gly⁸Arg^{26,34}Lys³⁶-GLP-1 (7-37);
Gly⁸Arg²⁶Lys³⁶-GLP-1(7-37); Gly⁸Arg³⁴Lys³⁶-GLP-1(7-37);

In a further preferred embodiment, the present invention relates to a GLP-1 derivative wherein the parent peptide is selected from the group comprising Arg²⁶-GLP-1(7-37), Arg³⁴-GLP-1 (7-37), Lys³⁶-GLP-1(7-37), Arg^{26,34}Lys³⁶-GLP-1(7-37), Arg²⁶Lys³⁶-GLP-1 (7-37), Arg³⁴Lys³⁶-GLP-1 (7-37), Gly⁸Arg²⁶-GLP-1 (7-37), Gly⁸Arg³⁴-GLP-1 (7-37), Gly⁸Lys³⁶-GLP-1(7-37), Gly⁸Arg^{26,34}Lys³⁶-GLP-1 (7-37), Gly⁸Arg²⁶Lys³⁶-GLP-1 (7-37) and Gly⁸Arg³⁴Lys³⁶-GLP-1 (7-37).

In a further preferred embodiment, the present invention relates to a GLP-1 derivative wherein the parent peptide is:
Arg²⁶-GLP-1 (7-37); Arg³⁴-GLP-1 (7-37); Arg^{26,34}Lys³⁶-GLP-1 (7-37);
Gly⁸Arg²⁶-GLP-1 (7-37); Gly⁸Arg³⁴-GLP-1 (7-37); Gly⁸Arg^{26,34}Lys³⁶-GLP-1 (7-37);
Val⁸Arg²⁶-GLP-1 (7-37); Val⁸Arg³⁴-GLP-1 (7-37); Val⁸Arg^{26,34}Lys³⁶-GLP-1(7-37);
Ser⁸Arg²⁶-GLP-1 (7-37); Ser⁸Arg³⁴-GLP-1 (7-37); Ser⁸Arg^{26,34}Lys³⁶-GLP-1 (7-37);
Thr⁸Arg²⁶-GLP-1 (7-37); Thr⁸Arg³⁴-GLP-1 (7-37);Thr⁸Arg^{26,34}Lys³⁶-GLP-1 (7-37);
Val⁸Glu³⁶Arg^{26,34}Lys³⁷GLP-1 (7-37); Val⁸Glu³⁶Arg^{26,34}Lys³⁷GLP-1(7-37);
Val⁸Asp³⁶Arg^{26,34}Lys³⁷GLP-1 (7-37); Val⁸Asp³⁶Arg^{26,34}Lys³⁷GLP-1(7-37);
Ser⁸Glu³⁶Arg^{26,34}Lys³⁷GLP-1(7-37); Ser⁸Glu³⁶Arg^{26,34}Lys³⁷GLP-1 (7-37);
Ser⁸Asp³⁶Arg^{26,34}Lys³⁷GLP-1 (7-37); Ser⁸Asp³⁶Arg^{26,34}Lys³⁷GLP-1 (7-37);
Thr⁸Glu³⁶Arg^{26,34}Lys³⁷GLP-1(7-37); Thr⁸Glu³⁶Arg^{26,34}Lys³⁷GLP-1 (7-37);
Thr⁸Asp³⁶Arg^{26,34}Lys³⁷GLP-1(7-37); Thr⁸Asp³⁶Arg^{26,34}Lys³⁷GLP-1 (7-37);
Gly⁸Glu³⁶Arg^{26,34}Lys³⁷GLP-1(7-37); Gl⁸Glu³⁶Arg^{26,34}Lys³⁷GLP-1 (7-37);
Gly⁸Asp³⁶Arg^{26,34}Lys³⁷GLP-1(7-37); Gly⁸Asp³⁶Arg^{26,34}Lys³⁷GLP-1 (7-37);
Arg^{26,34}Lys¹⁸GLP-1(7-37); Gly⁸Asp¹⁹Arg^{26,34}Lys¹⁸GLP-1(7-37);
Arg^{26,34}Lys²³GLP-1(7-37); Gly⁸Asp²⁴Arg^{26,34}Lys²³GLP-1(7-37);
Arg^{26,34}Lys²⁷GLP-1(7-37); Gly⁸Asp²⁸Arg^{26,34}Lys²⁷GLP-1(7-37);
Arg^{26,34}Lys¹⁸GLP-1(7-37); Val⁸Asp¹⁹Arg^{26,34}Lys¹⁸GLP-1(7-37);
Arg^{26,34}Lys²³GLP-1(7-37); Val⁸Asp²⁴Arg^{26,34}Lys²³GLP-1(7-37);
Arg^{26,34}Lys²⁷GLP-1(7-37); Val⁸Asp²⁸Arg^{26,34}Lys²⁷GLP-1(7-37);
Arg^{26,34}Lys¹⁸GLP-1(7-37); Ser⁸Asp¹⁹Arg^{26,34}Lys¹⁸GLP-1(7-37);
Arg^{26,34}Lys²³GLP-1(7-37); Ser⁸Asp²⁴Arg^{26,34}Lys²³GLP-1(7-37);
Arg^{26,34}Lys²⁷GLP-1(7-37); Ser⁸Asp²⁸Arg^{26,34}Lys²⁷GLP-1(7-37);
Arg^{26,34}Lys¹⁸GLP-1(7-37); Thr⁸Asp¹⁹Arg^{26,34}Lys¹⁸GLP-1(7-37);
Arg^{26,34}Lys²³GLP-1(7-37); Thr⁸Asp²⁴Arg^{26,}Lys²³GLP-1(7-37);
Arg^{26,34}Lys²⁷GLP-1(7-37); Thr⁸Asp²⁸Arg^{26,34}Lys²⁷GLP-1(7-37);

In a further preferred embodiment, the present invention relates to a GLP-1 derivative wherein the parent peptide is:
Arg²⁶Lys³⁶-GLP-1 (7-37); Arg³⁴Lys³⁶-GLP-1(7-37); Arg²⁶Lys³⁷-GLP-1(7-37); Arg³⁴Lys³⁷-GLP-1(7-37);
Arg²⁶Lys¹⁸GLP-1(7-37); Arg³⁴Lys¹⁸GLP-1(7-37); Arg²⁶Lys²³GLP-1(7-37); Arg³⁴Lys²³GLP-1(7-37);
Arg²⁶Lys²⁷GLP-1(7-37); Arg³⁴Lys²⁷GLP-1(7-37); Arg^{26,34}Lys¹⁸GLP-1(7-37); Arg^{26,34}Lys^{18,37}GLP-1 (7-37); Arg^{26,34}Lys²³GLP-1(7-37); Arg^{26,34}Lys^{23,37}GLP-1(7-37); Arg^{26,34}Lys²⁷GLP-1(7-37);
Arg^{26,34}Lys^{27,37}GLP-1(7-37);
Gly⁸GLP-1(7-37);Gly⁸Arg²⁶Lys³⁶-GLP-1(7-37);Gly⁸Arg³⁴Lys³⁶-GLP-1(7-37);Gly⁸Arg²⁶Lys³⁷-GLP-1(7-37);Gly⁸Arg³⁴Lys³⁷-GLP-1(7-37); Gly⁸Arg²⁶Lys²⁸GLP-1(7-37); Gly⁸Arg³⁴Lys¹⁸GLP-1(7-37);
Gly⁸Arg²⁶Lys²⁶GLP-1(7-37); Gly⁸Arg³⁴Lys²³GLP-1(7-37); Gly⁸Arg²⁶Lys²⁷GLP-1(7-37);
Gly⁸Arg³⁴Lys²⁷GLP-1(7-37); Gly⁸Arg²⁶Lys¹⁸GLP-1(7-37); Gly⁸Arg^{26,34}Lys^{18,37}GLP-1(7-37);
Gly⁸Arg^{26,34}Lys²³GLP-1(7-37); Gly⁸Arg^{26,34}Lys^{23,37}GLP-1(7-37); Gly⁸Arg^{26,34}Lys²⁷GLP-1(7-37);
Gly⁸Arg^{26,34}Lys^{27,37}GLP-1(7-37);
Val⁸GLP-1(7-37); Val⁸Arg²⁶Lys³⁶-GLP-1(7-37); Val⁸Arg³⁴Lys³⁶-GLP-1(7-37); Val⁸Arg²⁶Lys³⁷-GLP-1(7-37); Val⁸Arg³⁴Lys³⁷-GLP-1(7-37); Val⁸Arg²⁶Lys¹⁸GLP-1(7-37); Val⁸Arg³⁴Lys¹⁸GLP-1(7-37);
Val⁸Arg²⁶Lys²³GLP-1(7-37); Val⁸Arg³⁴Lys²³GLP-1(7-37); Val⁸Arg²⁶Lys²⁷GLP-1(7-37);
Val⁸Arg³⁴Lys²⁷GLP-1(7-37); Val⁸Arg^{26,34}Lys¹⁸GLP-1(7-37); Val⁸Arg^{26,34}Lys^{18,37}GLP-1(7-37);
Val⁸Arg^{26,34}Lys²³GLP-1(7-37); Val⁸Arg^{26,34}Lys^{23,37}GLP-1(7-37); Val⁸Arg^{26,34}Lys²⁷GLP-1(7-37);
Val⁸Arg^{26,34}Lys^{27,37}GLP-1(7-37);

The total number of different amino acids between the derivative of the GLP-1 analog and the corresponding native form of GLP-1 does not exceed six. Preferably, the number of different amino acids is five. More preferably, the number of different amino acids is four. Even more preferably, the number of different amino acids is three. Even more preferably, the number of different amino acids is two. Most preferably, the number of different amino acids is one. In order to determine the number of different amino acids, one should compare the amino acid sequence of the derivative of the GLP-1 analog of the present invention with the corresponding native GLP-1. For example, there are two different amino acids (at positions 8 and 26) between the derivative Gly⁸Arg²⁶Lys³⁴(N^{ε}-(7-deoxycholoyl))-GLP-1(7-40) and the corresponding native GLP-1 (i.e., GLP-1 (7-40)). Similarly, there is only one different amino acid (at position 34) between the derivative Lys²⁶(N^{ε}-(7-deoxycholoyl))Arg³⁴-GLP-1(7-40) and the corresponding native GLP-1.

The derivatives of the GLP-1 analogs of the present invention preferably have only one or two Lys. The ε-amino group of one or both Lys is substituted with a lipophilic substituent, Preferably, the derivatives of the GLP-1 analogs of the present invention have only one Lys. In a more preferred embodiment, there is only one Lys which is located at the carboxy terminus of the derivative of the GLP-1 analogs. In an even more preferred embodiment, the derivatives of the GLP-1 analogs of the present invention have only one Lys and Glu or Asp is adjacent to Lys.

### Derivatives

The term "derivative" is defined as a modification of one or more amino acid residues of a peptide by chemical means, either with or without an enzyme, *e.g*. by alkylation, acylation, ester formation or amide formation.

### Modified Histidine

In the GLP-1 derivatives of the present invention, the histidine residue at position 7 is modified. Generally the histidine residue at position 7 may be replaced with a group A wherein
A is: 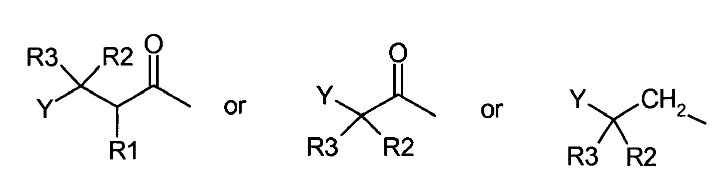 wherein R¹, R² and R³ are independently H, lower alkyl, optionally substituted phenyl, NH₂, NH-CO-(lower alkyl), -OH, lower alkoxy, halogen, SO₂-(lower alkyl) or CF₃, wherein said phenyl is optionally substituted with at least one group selected from NH₂, -OH, lower alkyl, lower alkoxy, halogen, SO₂-(lower alkyl), NH-CO-(lower alkyl) or CF₃, or R¹ and R² may together form a bond; and Y is a five or six membered ring system selected from the group consisting of: 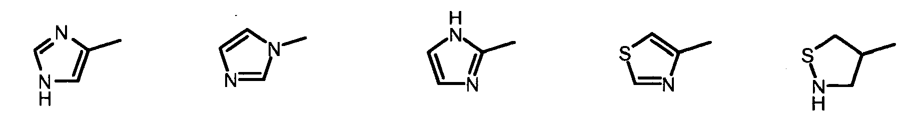 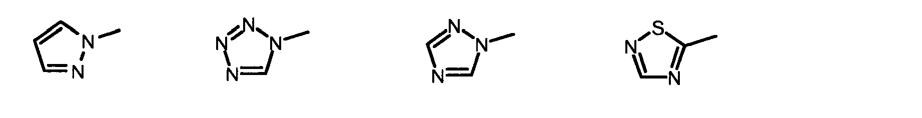 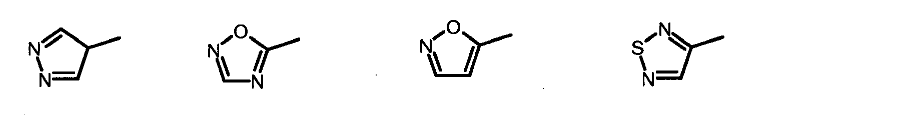 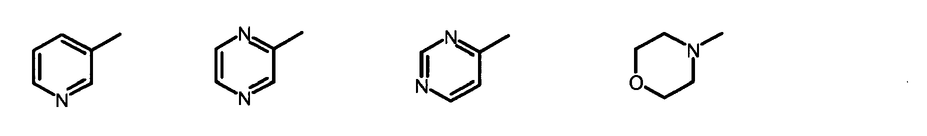 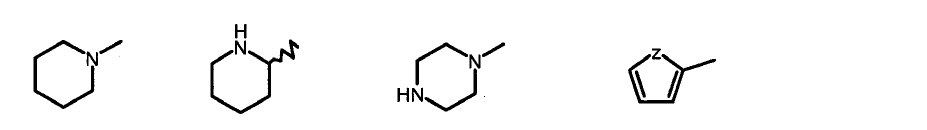 wherein Z is N, O or S, and said ring system is optionally substituted with one or more functional groups selected from the group consisting of NH₂, NO₂, OH, lower alkyl, lower alkoxy, halogen, CF₃ and aryl (i.e. optionally substituted phenyl as defined above), provided that A is not histidine.

The terms "lower alkyl" and "lower alkoxy" refer to an alkyl or alkoxy group, respectively, having 1-6 carbon atoms.

Accordingly to the present invention, A is: 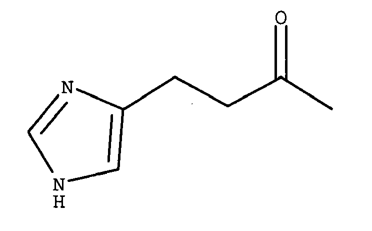 , or 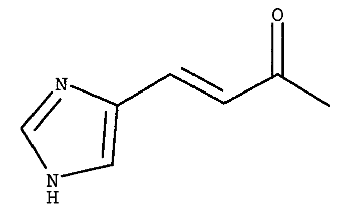 , or 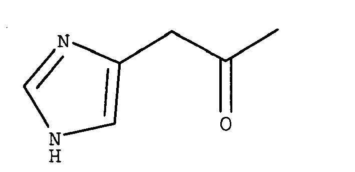 , or 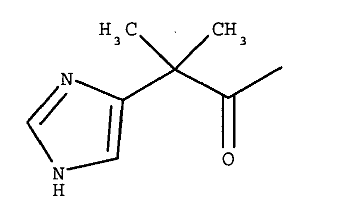 , or

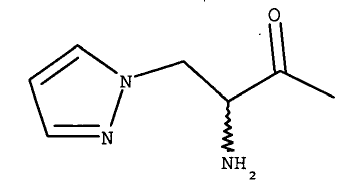

In another preferred embodiment, A is 4-imidazopropionyl.

In another preferred embodiment, A is 4-imidazoacetyl.

In another preferred embodiment, A is 4-imidazo-α,α-dimethyl-acetyl.

In another preferred embodiment the GLP-1 derivatives of formula II may be selected from
desamino-His⁷,Arg⁷,Lys³⁴ (N^{ε}-(γ-glutamyl(N^{α}-hexadecanoyl))) GLP-1 (7-37)-OH,
desamino-His⁷,Arg²⁶,Lys³⁴ (N^{ε}-(γ-glutamyl(N^{α}-octanoyl))) GLP-1 (7-37)-OH,
desamino-His⁷,Arg³⁴, Lys²⁶(N^{ε}-(γ-glutamyl(N^{α}-hexadecanoyl))) GLP-1 (7-37),
desamino-His⁷,Arg³⁴,Lys²⁶(N^{ε}-(γ-aminobutyroyl(N^{γ}-hexadecanoyl))) GLP-1 (7-37),
desamino-His⁷,Arg³⁴, Lys²⁶(N^{ε}-(β-alanyl(N^{γ}-hexadecanoyl))) GLP-1 (7-37),
Arg³⁴,Ala⁸(N^{α}-(imidazol-4-ylprop-2-enoyl),Lys³⁶(N^{ε}-(γ-aminobutyroyl(N^{γ}-hexadecanoyl))) GLP-1 (8-37),
Arg³⁴,Ala⁸(N^{α}-(imidazol-4-ylacetyl),Lys²⁶(N^{ε}-(γ-aminobutyroyl(N^{γ}-hexadecanoyl))) GLP-1 (8-37),
desamino-His⁷,Arg³⁴, Lys²⁶(N^{ε}-(γ-aminobutyroyl(N^{γ}-tetradecanoyl))) GLP-1 (7-37),
desamino-His⁷,Arg³⁴, Lys²⁶(N^{ε}-(γ-aminobutyroyl(N^{γ}-octadecanoyl))) GLP-1 (7-37).

### Lipophilic Substituents

To obtain a satisfactory protracted profile of action of the GLP-1 derivative, the lipophilic substituents attached to the parent GLP-1 peptide comprises from 12-24 carbon atoms and most preferably 12-18 carbon atoms. A lipophilic substituent may be attached to an amino group of the parent GLP-1 peptide by means of a carboxyl group of the lipophilic substituent which forms an amide bond with an amino group of the amino acid residue to which it is attached.

In a preferred embodiment, the GLP-1 derivatives of the present invention have three lipophilic substituents.

In a more preferred embodiment, the GLP-1 derivatives of the present invention have two lipophilic substituents.

In an even more preferred embodiment, the GLP-1 derivatives of the present invention have one lipophilic substituent.

Each lipophilic substituent can be attached to (a) the free amino group of the N-terminal amino acid, (b) the free carboxy group of the C-terminal amino acid, (c) the ε-amino group of Lys and/or (d) the carboxy group which is part of the R group of Asp or Glu.

In a preferred embodiment, a lipophilic substituent is attached to only the carboxy group which is part of the R group of Asp or Glu.

In a preferred embodiment, a lipophilic substituent is attached to only the free carboxy group of the C-terminal amino acid.

In another preferred embodiment, a lipophilic substituent is attached to only an ε-amino group of Lys.

According to the invention, the lipophilic substituent is attached to the parent GLP-1 peptide by means of a spacer, e.g. in such a way that a carboxyl group of the spacer forms an amide bond with an amino group of the parent GLP-1 peptide. In a preferred embodiment, the spacer is an α,ω-amino acid. Examples of suitable spacers are succinic acid, Lys, Glu or Asp, or a dipeptide such as Gly-Lys. When the spacer is succinic acid, one carboxyl group thereof may form an amide bond with an amino group of the amino acid residue, and the other carboxyl group thereof may form an amide bond with an amino group of the lipophilic substituent. When the spacer is Lys, Glu or Asp, the carboxyl group thereof may form an amide bond with an amino group of the amino acid residue, and the amino group thereof may form an amide bond with a carboxyl group of the lipophilic substituent. When Lys is used as the spacer, a further spacer may in some instances be inserted between the ε-amino group of Lys and the lipophilic substituent. In one preferred embodiment, such a further spacer is succinic acid which forms an amide bond with the ε-amino group of Lys and with an amino group present in the lipophilic substituent. In another preferred embodiment such a further spacer is Glu or Asp which forms an amide bond with the ε-amino group of Lys and another amide bond with a carboxyl group present in the lipophilic substituent. Other preferred spacers are γ-L-glutamyl, β-L-asparagyl, glycyl, β-L-alanyl, and α-(γ-aminobutanoyl).

In another preferred embodiment of the present invention, the lipophilic substituent has a group which can be negatively charged. One preferred group which can be negatively charged is a carboxylic acid group.

In a further preferred embodiment, the lipophilic substituent is attached to the parent peptide by means of a spacer which is an unbranched alkane α,ω-dicarboxylic acid group having from 1 to 7 methylene groups, preferably two methylene groups which spacer forms a bridge between an amino group of the parent peptide and an amino group of the lipophilic substituent.

In a further preferred embodiment, the lipophilic substituent is attached to the parent peptide by means of a spacer which is an amino acid residue except Cys or Met, or a dipeptide such as Gly-Lys. In the present text, the phrase "a dipeptide such as Gly-Lys" means a dipeptide wherein the C-terminal amino acid residue is Lys, His or Trp, preferably Lys, and wherein the N-terminal amino acid residue is selected from the group comprising Ala, Arg, Asp, Asn, Gly, Glu, Gln, Ile, Leu, Val, Phe and Pro.

In a further preferred embodiment, the lipophilic substituent is attached to the parent peptide by means of a spacer which is an amino acid residue except Cys or Met, or is a dipeptide such as Gly-Lys and wherein an amino group of the parent peptide forms an amide bond with a carboxylic group of the amino acid residue or dipeptide spacer, and an amino group of the amino acid residue or dipeptide spacer forms an amide bond with a carboxyl group of the lipophilic substituent.

In a further preferred embodiment, the lipophilic substituent comprises a partially or completely hydrogenated cyclopentanophenathrene skeleton.

In a further preferred embodiment, the lipophilic substituent is a straight-chain or branched alkyl group.

In a further preferred embodiment, the lipophilic substituent is an acyl group of a straight-chain or branched fatty acid.

In a further preferred embodiment, the lipophilic substituent is an acyl group selected from the group comprising CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-. In a most preferred embodiment, the lipophilic substituent is tetradecanoyl. In another most preferred embodiment, the lipophilic substituent is hexadecanoyl.

In a further preferred embodiment, the lipophilic substituent is an acyl group of a straight-chain or branched alkane α,ωdicarboxylic acid.

In a further preferred embodiment, the lipophilic substituent is an acyl group selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In a further preferred embodiment, the lipophilic substituent with the attached spacer is a group of the formula CH₃(CH₂)ₚNH-CO(CH₂)₂CO-, wherein p is an integer of from 8 to 33, preferably from 12 to 28.

In a further preferred embodiment, the lipophilic substituent with the attached spacer is a group of the formula CH₃(CH₂)ᵣCO-NHCH(COOH)(CH₂)₂CO-, wherein r is an integer of from 10 to 24.

In a further preferred embodiment, the lipophilic substituent with the attached spacer is a group of the formula CH₃(CH₂)ₛCO-NHCH((CH₂)₂COOH)CO-, wherein s is an integer of from 8 to 24.

In a further preferred embodiment, the lipophilic substituent with the attached spacer is a group of the formula -NHCH(COOH)(CH₂)₄NH-CO(CH₂)ᵤCH₃, wherein u is an integer of from 8 to 18.

In a further preferred embodiment, the lipophilic substituent with the attached spacer is a group of the formula CH₃(CH₂)ᵥCO-NH-(CH₂)_{z}-CO, wherein n is an integer of from 8 to 24 and z is an integer of from 1 to 6.

In a further preferred embodiment, the lipophilic substituent with the attached spacer is a group of the formula -NHCH(COOH)(CH₂)₄NH-COCH((CH₂)₂COOH)NH-CO(CH₂)_{w}CH₃, wherein w is an integer of from 10 to 16.

In a further preferred embodiment, the lipophilic substituent with the attached spacer is a group of the formula -NHCH(COOH)(CH₂)₄NH-CO(CH₂)₂CH(COOH)NH-CO(CH₂)ₓCH₃, wherein x is an integer of from 10 to 16.

In a further preferred embodiment, the lipophilic substituent with the attached spacer is a group of the formula -NHCH(COOH)(CH₂)₄NH-CO(CH₂)₂CH(COOH)NHCO(CH₂)_{y}CH₃, wherein y is zero or an integer of from 1 to 22.

In a further preferred embodiment, the lipophilic substituent can be negatively charged. Such a lipophilic substituent can for example be a substituent which has a carboxyl group.

### Other Derivatives

The derivatives of GLP-1 analogues of the present invention may be in the form of one or more of (a) a C-1-6-ester, (b) an amide, C-1-6-alkylamide, or C-1-6-dialkylamide, and (c) a pharmaceutical salt. In a preferred embodiment, the derivatives of GLP-1 analogues are in the form of an acid addition salt or a carboxylate salt, most preferably in the form of an acid addition salt.

### Pharmaceutical Compositions

The present invention also relates to pharmaceutical compositions comprising a derivative of a GLP-1 analog of the present invention and a pharmaceutically acceptable vehicle or carrier.

Preferably, the pharmaceutical compositions comprise an isotonic agent, a preservative and a buffer. Examples of isotonic agents are sodium chloride, mannitol and glycerol. Examples of preservatives are phenol, m-cresol, methyl p-hydroxybenzoate and benzyl alcohol. Suitable buffers include sodium acetate, sodium citrate, glycylglycine, histidine, 2-phenylethanol and sodium phosphate.

The pharmaceutical compositions preferably further comprise a surfactant in order to improve the solubility and/or the stability of the GLP-1 derivative. Preferably, the surfactant is poloxymer 188, tween 20 or tween 80.

The pharmaceutical compositions preferably also comprise zinc.

The pharmaceutical compositions preferably also comprise protamine.

The pharmaceutial compositions preferably further comprise another antidiabetic agent. The term "antidiabetic agent" includes compounds for the treatment and/or prophylaxis of insulin resistance and diseases wherein insulin resistance is the pathophysiological mechanism.

In one embodiment of this invention, the antidiabetic agent is an insulin, more preferably human insulin.

In another embodiment the antidiabetic agent is a hypoglycaemic agent, preferably an oral hypoglycaemic agent. Oral hypoglycaemic agents are preferably selected from the group consisting of sulfonylureas, biguanides, thiazolidinediones, glucosidase inhibitors, glucagon antagonists, GLP-1 agonists, potasium channel openers, insulin sensitizers, hepatic enzyme inhibitors, glucose uptake modulators, compounds modifying the lipid metabolism, compounds lowering food intake, and agents acting on the ATP-dependent potassium channel of the β-cells. Preferred sulfonylureas are tolbutamide, glibenclamide, glipizide and gliclazide. A preferred biguanide is metformin. Preferred thiazolidinediones are troglitazone and ciglitazone. A preferred glucosidase inhibitor is acarbose. Preferred agents acting on the ATP-dependent potassium channel of the β-cells are: glibenclamide, glipizide, gliclazide, and repaglinide.

In a preferred embodiment of the present invention, the GLP-1 derivative is provided in the form of a composition suitable for administration by injection. Such a composition can either be an injectable solution ready for use or it can be an amount of a solid composition, *e.g*. a lyophilised product, which has to be dissolved in a solvent before it can be injected. The injectable solution preferably contains not less than about 2 mg/ml, preferably not less than about 5 mg/ml, more preferred not less than about 10 mg/ml of the GLP-1 derivative and, preferably, not more than about 100 mg/ml of the GLP-1 derivative.

The pharmaceutical compositions of the present invention also preferably comprise another anti-obesity drug.

In one embodiment of this invention, the antiobesity agent is leptin.

In another embodiment the antiobesity agent is amphetamin.

In another embodiment the antiobesity agent is dexfenfluramine.

In another embodiment the antiobesity agent is sibutramine.

In another embodiment the antiobesity agent is orlistat.

In another embodiment the antiobesity agent is selected from a group of CART agonists, NPY antagonists, orexin antagonists, H3-antagonists, TNF agonists, CRF agonists, CRF BP antagonists, urocortin agonists, β3 agonists, MSH agonists, CCK agonists, serotonin re-uptake inhibitors, mixed serotonin and noradrenergic compounds, 5HT agonists, bombesin agonists, galanin antagonists, growth hormone, growth hormone releasing compounds, glucagon, TRH agonists, uncoupling protein 2 or 3 modulators, leptin agonists, DA agonists (Bromocriptin, Doprexin), lipase/amylase inhibitors, PPAR modulators, PXR modulators or TR β agonists.

A number of GLP-1 derivatives of the present invention exist in a partially structured micellar-like aggregated form when added to water or an aqueous solution. This structure makes them more soluble and stable in solution as compared to native GLP-1. The increased solubility and stability can be seen by comparing the solubility after 9 days of standing for a derivative and normal GLP-1 (7-37) in a pharmaceutical formulation, e.g. 5 mM phosphate buffer, pH 6.9 added 0.1 M NaCl.

Circular Dichroism (CD) can be used to show that the GLP-1 derivatives have a certain partially structured conformation. In contrast to native GLP-1 (7-37) the helix content of some GLP-1 derivatives of the present invention increases with increasing concentration, from 10-15% to 30-35% (at a concentration of 500 µM) in parallel with peptide self-association. For the GLP-1 derivatives forming partially structured micellar-like aggregates in aqueous solution the helix content remains constant above 30% at concentrations of 10 µM.

The size of the partially helical, micelle-like aggregates may be estimated by size-exclusion chromatography. Similarly, the apparent (critical micelle concentrations) CMC's of the peptides may be estimated from the concentration dependent fluorescence in the presence of appropriate dyes (e.g. Brito, R. & Vaz, W. (1986) Anal. Biochem. 152, 250-255).

Thus, the present invention also relates to pharmaceutical compositions comprising water and a GLP-1 derivative of the present invention which has a helix content as measured by Circular Dichroism at 222 nm in H₂O at 22 ± 2°C exceeding 25%, preferably in the range of 25% to 50%, at a peptide concentration of about 10 µM.

### Uses

The present invention also relates to the use of a GLP-1 derivative of the present invention for the preparation of a medicament which has a protracted profile of action relative to GLP-1(7-37).

The present invention also relates to the use of a GLP-1 derivative of the present invention for the preparation of a medicament with protracted effect for the treatment of non-insulin dependent diabetes mellitus.

The present invention also relates to the use of a GLP-1 derivative of the present invention for the preparation of a medicament with protracted effect for the treatment of insulin dependent diabetes mellitus.

The present invention also relates to the use of a GLP-1 derivative of the present invention for the preparation of a medicament to treat insulin resistance.

The present invention also relates to the use of a GLP-1 derivative of the present invention for the preparation of a medicament with protracted effect for the treatment of obesity.

The present invention also discloses a method of treating insulin dependent or non-insulin dependent diabetes mellitus in a patient in need of such a treatment, comprising administering to the patient a therapeutically effective amount of a GLP-1 derivative of the present invention together with a pharmaceutically acceptable carrier.

The present invention also discloses a method of treating obesity in a patient in need of such a treatment, comprising administering to the patient a therapeutically effective amount of a GLP-1 derivative of the present invention together with a pharmaceutically acceptable carrier.

The particular GLP-1 derivative to be used and the optimal dose level for any patient will depend on the disease to be treated and on a variety of factors including the efficacy of the specific peptide derivative employed, the age, body weight, physical activity, and diet of the patient, on a possible combination with other drugs, and on the severity of the case.

The pharmaceutical compositions of the present invention may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of the GLP-1 derivative in the form of a nasal or pulmonal spray. As a still further option, the GLP-1 derivatives of the invention can also be administered transdermally, *e.g*. from a patch, optionally a iontophoretic patch, or transmucosally, *e.g.* bucally.

### Methods of Production

The parent peptide can be produced by a method which comprises culturing a host cell containing a DNA sequence encoding the polypeptide and capable of expressing the polypeptide in a suitable nutrient medium under conditions permitting the expression of the peptide, after which the resulting peptide is recovered from the culture.

The medium used to culture the cells may be any conventional medium suitable for growing the host cells, such as minimal or complex media containing appropriate supplements. Suitable media are available from commercial suppliers or may be prepared according to published recipes (*e.g.* in catalogues of the American Type Culture Collection). The peptide produced by the cells may then be recovered from the culture medium by conventional procedures including separating the host cells from the medium by centrifugation or filtration, precipitating the proteinaceous components of the supernatant or filtrate by means of a salt, *e.g*. ammonium sulphate, purification by a variety of chromatographic procedures, *e.g*. ion exchange chromatography, gel filtration chromatography, affinity chromatography, or the like, dependent on the type of peptide in question.

The DNA sequence encoding the parent peptide may suitably be of genomic or cDNA origin, for instance obtained by preparing a genomic or cDNA library and screening for DNA sequences coding for all or part of the peptide by hybridisation using synthetic oligonucleotide probes in accordance with standard techniques (see, for example, Sambrook, J, Fritsch, EF and Maniatis, T, Molecular Cloning: A Laboratory Manual, Cold Spring Harbor Laboratory Press, New York, 1989). The DNA sequence encoding the peptide may also be prepared synthetically by established standard methods, *e.g*. the phosphoamidite method described by Beaucage and Caruthers, Tetrahedron Letters 22 (1981), 1859 - 1869, or the method described by Matthes et al., EMBO Journal 3 (1984), 801 - 805. The DNA sequence may also be prepared by polymerase chain reaction using specific primers, for instance as described in US 4,683,202 or Saiki et al., Science 239 (1988), 487 - 491.

The DNA sequence may be inserted into any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, *e.g*. a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the peptide is operably linked to additional segments required for transcription of the DNA, such as a promoter. The promoter may be any DNA sequence which shows transcriptional activity in the host cell of choice and may be derived from genes encoding proteins either homologous or heterologous to the host cell. Examples of suitable promoters for directing the transcription of the DNA encoding the peptide of the invention in a variety of host cells are well known in the art, cf. for instance Sambrook *et al., supra.*

The DNA sequence encoding the peptide may also, if necessary, be operably connected to a suitable terminator, polyadenylation signals, transcriptional enhancer sequences, and translational enhancer sequences. The recombinant vector of the invention may further comprise a DNA sequence enabling the vector to replicate in the host cell in question.

The vector may also comprise a selectable marker, *e.g*. a gene the product of which complements a defect in the host cell or one which confers resistance to a drug, *e.g*. ampicillin, kanamycin, tetracyclin, chloramphenicol, neomycin, hygromycin or methotrexate.

To direct a parent peptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the peptide in the correct reading frame. Secretory signal sequences are commonly positioned 5' to the DNA sequence encoding the peptide. The secretory signal sequence may be that normally associated with the peptide or may be from a gene encoding another secreted protein.

The procedures used to ligate the DNA sequences coding for the present peptide, the promoter and optionally the terminator and/or secretory signal sequence, respectively, and to insert them into suitable vectors containing the information necessary for replication, are well known to persons skilled in the art (cf., for instance, Sambrook *et al., supra*)*.*

The host cell into which the DNA sequence or the recombinant vector is introduced may be any cell which is capable of producing the present peptide and includes bacteria, yeast, fungi and higher eukaryotic cells. Examples of suitable host cells well known and used in the art are, without limitation, *E. coli, Saccharomyces cerevisiae,* or mammalian BHK or CHO cell lines.

The GLP-1 derivatives and analogues of the present invention may be prepared by methods known *per se* in the art. For example, the polypeptide portion may be prepared by chemical synthesis using solid phase protein synthesis techniques, or using recombinant DNA techniques, and the GLP-1 peptide having attached thereto a lipophilic substituent may e.g. be prepared as described in WO 98/08871. Coupling of the group A, comprising the 5- or 6-membered ring system Y, to the N-terminal end of the peptide may similarly be performed using solid phase protein synthesis techniques analogous to the addition of a natural amino acid to the N-terminal end of a peptide, or alternatively, by means of an activated ester. Methods suitable for the addition of unsaturated, partially saturated or saturated heterocyclyl-containing groups, e.g. heteroaryl groups such as imidazole groups, to the N-terminal end of a GLP-1 peptide are further described in EP 0708179-A2.

The pharmaceutical compositions of the present invention may be prepared by conventional techniques, *e.g*. as described in Remington's Pharmaceutical Sciences, 1985 or in Remington: The Science and Practice of Pharmacy, 19th edition, 1995.

For example, injectable compositions of the GLP-1 derivative of the invention can be prepared using the conventional techniques of the pharmaceutical industry which involves dissolving and mixing the ingredients as appropriate to give the desired end product.

According to one procedure, the GLP-1 derivative is dissolved in an amount of water which is somewhat less than the final volume of the composition to be prepared. An isotonic agent, a preservative and a buffer is added as required and the pH value of the solution is adjusted - if necessary - using an acid, *e.g*. hydrochloric acid, or a base, *e.g*. aqueous sodium hydroxide as needed. Finally, the volume of the solution is adjusted with water to give the desired concentration of the ingredients.

A composition for nasal administration of certain peptides may, for example, be prepared as described in European Patent No. 272097 (to Novo Nordisk A/S) or in WO 93/18785.

The present invention also discloses methods for producing a GLP-1 derivative of the present invention, comprising alkylating, acylating and/or amidating the corresponding GLP-1 analog.

The present invention is further illustrated by the following examples which, however, are not to be construed as limiting the scope of protection. The features disclosed in the foregoing description and in the following examples may, both separately and in any combination thereof, be material for realising the invention in diverse forms thereof.

### EXAMPLES

The examples below illustrate the preparation of modified GLP-1 derivatives according to the present invention. In each case, the basic peptide to be modified may, for example, comprise the amino acid residues of GLP-1 (8-36), GLP-1 (8-37) or GLP-1 (8-38). The peptide may of course also contain other modifications as described above.

The following acronyms for commercially available chemicals are used:
- DMF :: N,N-Dimethylformamide.
- DCC :: N,N-Dicyclohexylcarbodiimide
- NMP :: N-Methyl-2-pyrrolidone.
- EDPA :: N-Ethyl-N,N-diisopropylamine.
- TFA :: Trifluoroacetic acid.
- THF :: Tetrahydrofuran.
- Pal-ONSu:: Hexadecanoic acid 2,5-dioxopyrrolidin-1-yl ester.
- N^{α}-alkanoyl-Glu(ONSu)-OBu^{t}:: N^{α}-Alkanoyl-(L)-glutamic acid α-t-butyl-γ-2,5- dioxopyrrolidin-1-yl diester.
- N^{α}-Pal-γ-Glu(ONSu)-OBu^{t}:: N^{α}-Hexadecanoyl-(L)-glutamic acid α-t-butyl-γ-2,5- dioxopyrrolidin-1-yl diester.
- N^{α}-Ste-γ-Glu (ONSu)-OBu^{t}:: N^{α}-Octadecanoyl-(L)-glutamic acid α-t-butyl-γ2,5-dioxopyrrolidin- 1-yl diester.

### Abbreviations:

PDMS: Plasma Desorption Mass Spectrometry
HPLC: High Performance Liquid Chromatography
amu: atomic mass units

### EXAMPLE 1

### General method A:

### Synthesis of alkanoic acid 2,5-dioxopyrrolidin-1-yl ester:

To a solution of the alkanoic acid (34.7 mmol) and N-hydroxysuccinimide (4 g, 34.7 mmol) in anhydrous acetonitril (10 ml) was added a solution of DCC (7.15 g, 34.7 mmol) in anhydrous dichloromethane (15 ml), and the resulting reaction mixture was stirred for 16 h at room temperature. The precipitated solid was filtered off and recrystallised from a mixture of n-heptane and 2-propanol. The precipitate was dried *in vacuo* for 16 h to give the title compound.

### Synthesis of Lys(N^{ε}-alkanoyl)-peptide:

To a mixture of the peptide (5.9 µmol), EDPA (21 mg, 164 µmol), NMP (5.8 ml) and water (2.9 ml) was added a solution of the alkanoic acid 2,5-dioxopyrrolidin-1-yl ester (37 µmol), prepared as described above, in NMP (0.5 ml). The reaction mixture was gently shaken for 5 min at room temperature, and then allowed to stand for an additional 2 h at room temperature. The reaction was quenched by the addition of a solution of glycine (9.7 mg, 129 µmol) in water (97 µl). The solvent was removed *in vacuo,* and the residue was purified by column chromatography using a cyanopropyl column (Zorbax 300SB-CN) and a standard acetonitril/TFA system. The column was heated to 65°C and the acetonitril gradient was 0-100% for 60 minutes.

Coupling of a desired group A comprising the 5- or 6-membered ring system Y to the N-terminal end of the peptide may be performed using solid phase protein synthesis techniques as explained above.

### EXAMPLE 2

### General method B:

### Synthesis of N^{α}-alkanoyl-(L)-glutamic acid α-tert-butyl-γ-(2,5-dioxopyrrolidin-1-yl) diester:

A suspension of the alkanoic acid 2,5-dioxopyrrolidin-1-yl ester (16.2 mmol), prepared as described under General method A, (L)-glutamic acid α-tert-butyl ester (3.28 g, 16.2 mmol), DMF (268 ml) and EDPA (2.1 g, 16.2 mmol) was stirred for 64 h at room temperature. The reaction mixture was concentrated *in vacuo* and the residue was dissolved in ethyl acetate (50 ml). The resulting solution was washed with 5% aqueous citric acid (2x25 ml). The solvent was concentrated removed *in vacuo* and the residue dissolved in DMF (36 ml). The resulting solution was carefully added to a 10% aqueous solution of citric acid (357 ml) and extracted with ethyl acetate (200 ml) and dried (MgSO₄). The solvent was concentrated removed *in vacuo* to give the crude glutamic diester intermediate. To a mixture of the crude diester, N-hydroxysuccinimide (1.85 g, 16.1 mmol) and anhydrous DMF (25 ml) was added a solution of DCC (3.32 g, 16.1 mmol) in anhydrous dichloromethane (15 ml). The resulting mixture was stirred at ambient temperature for 20 h. The reaction mixture was filtered and the solvent was concentrated removed *in vacuo.* The residue was purified on a silica gel column (40-63 µm) and eluted with a mixture of dichloromethane and acetonitril (1:1) to give the title compound.

### Synthesis of Lys(N^{ε}-(γ-glutamyl(N^{α}-alkanoyl)))peptide

To a mixture of the peptide (4.2 µmol), EDPA (15.3 mg, 119 µmol), NMP (2 ml) and water (1 ml) was added a solution of N^{α}-alkanoyl-(L)-glutamic acid α-tert-butyl-γ-(2,5-dioxopyrrolidin-1-yl) diester (12.7 µmol), prepared as described above, in NMP (135 ml). The reaction mixture was gently shaken for 5 min at room temperature and then allowed to stand for an additional 2 h at room temperature. The reaction was quenched by the addition of a solution of glycine (7 mg, 93 µmol) in water (698 µl). A 0.5% aqueous solution of ammonium acetate (42 ml) was added, and the resulting mixture was eluted onto a Varian 5g C8 Mega Bond Elut^{®} cartridge, the immobilised compound was washed with 5% aqueous acetonitril (25 ml) and finally liberated from the cartridge by elution with TFA (25 ml). The eluate was concentrated *in vacuo,* and the residue was purified by column chromatography using a cyanopropyl column (Zorbax 300SB-CN) and a standard acetonitril/TFA system. The column iwa heated to 65°C and the acetonitril gradient is 0-100% for 60 minutes.

Coupling of a desired group A comprising the 5- or 6-membered ring system Y to the N-terminal end of the peptide may be performed using solid phase protein synthesis techniques as explained above.

### EXAMPLE 3

### Synthesis of N-terminal modified peptides

Peptides were synthesised according to the Fmoc strategy on an Applied Biosystems 431A peptide synthesiser in 0.25 mmol scale using the manufacturer supplied FastMoc UV protocols starting with a Fmoc-Gly-Wang resin (NovaBiochem). The protected amino acid derivatives used were commercially obtained Fmoc amino acids, and Adoc-Imidazolylpropionic acid. The derivatives used where side chain protection was needed were: Fmoc-Arg(Pmc), Fmoc-Trp(Boc), Fmoc-Glu(OBut), Fmoc-Lys(Boc), Fmoc-Gln(Trt), Fmoc-Tyr(But), Fmoc-Ser(But), Fmoc-Thr(But), Fmoc-His(Trt) and Fmoc-Asp(OBut), and Adoc-Imidazolylpropionic acid.

The peptides were cleaved from the resin and side chain deprotected in TFA/phenol/thioanisole/water/ethanedithiol (83.25:6.25:4.25:4.25:2.00) for 180 min. The cleavage mixture was filtered and the filtrate was concentrated by a stream of nitrogen. The crude peptide was precipitated from this oil with diethyl ether and washed twice with diethyl ether. After drying the crude peptide was dissolved in 50% aqueous acetic acid and diluted to 10% acetic acid with water and purified by semipreparative HPLC on a 25 mm x 250 mm column packed with 7 µm C-18 silica. The column was eluted with a gradient of acetonitril against 0.05 M (NH₄)₂SO₄, pH 2.5 at 10 ml/min. at 40°C. The peptide-containing fractions were collected, diluted with 3 volumes of water and applied to a Sep-Pak® C18 cartridge (Waters part. 51910) which was equilibrated with 0.1% TFA. The peptide was eluted from the Sep-Pak® cartridge with 70% acetonitrile/0.1 % TFA, water and isolated from the eluate by lyophilization after dilution with water. The final product obtained was characterised by amino acid analysis, analytical RP-HPLC and by PDMS. Amino acid analysis and mass spectrometry agreed with the expected structure within the experimental error of the method (mass spectrometry +/- 2 amu, amino acid analysis +/- 10%, RP-HPLC showed a peptide purity >95%).

The RP-HPLC analysis was performed using UV detection at 214 nm and a Vydac 218TP54 4.6 mm x 250 mm, 5 µm C-18 silica column which was eluted at 1 ml/min. at 42°C. Two different elution conditions were used: a gradient of 5-60% acetonitrile /0.1 M ammonium sulfate, water pH 2.5; and a gradient of 5-60% acetonitrile, 0.1 % TFA / 0.1 % TFA, water.

### Example 4

### Synthesis of desamino-His⁷,Arg²⁶,Lys³⁴ (N^{ε}-(γ-glutamyl(N^{α}-hexadecanoyl))) GLP-1 (7-37)-OH.

To a mixture of desamino-His⁷,Arg²⁶,Lys³⁴ GLP-1 (7-37)-OH (14.3 mg, 4.2 µmol), EDPA (15.3 mg, 119 µmol), NMP (2 ml) and water (1 ml) was added a solution of Pal-Glu(ONSu)-OBu^{t} (6.84 mg, 12.7 µmol) in NMP (171µl). The reaction mixture was gently shaken for 5 min. at room temperature, and then allowed to stand for an additional 50 min. at room temperature. The reaction was quenched by the addition of a solution of glycine (7 mg, 99 µmol) in water (700 µl). A 0.5 % aqueous solution of ammonium acetate (42 ml) was added, and the resulting mixture eluted onto a Varian 5g C8 Mega Bond Elut^{®}, the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (5.6 mg, 35 %) was isolated, and the product was analysed by PDMS. The m/z value for the protonated molecular ion was found to be 3738 +- 3. The resulting molecular weight is thus 3737 +- 3 amu (theoretical value 3737 amu).

### Example 5

### Synthesis of Cap-Glu(ONSu)-OBu^{t}.

To a solution of octanoic acid (5 g, 34.7 mmol) and N-hydroxysuccinimide (4 g, 34.7 mmol) in anhydrous acetonitril (10 ml) was added a solution of DCC (7.15 g, 34.7 mmol) in anhydrous dichloromethane (15 ml), and the resulting reaction mixture stirred for 16 h at room temperature. The precipitated solid was filtered off and recrystallised from a mixture of n-heptane (40 ml) and 2-propanol (2 ml). The precipitate was dried in a vacuum drying oven for 16 h to give the intermediate Cap-ONSu. A suspension of the crude ester intermediate (3.9 g, 16.2 mmol), (L)-H-Glu(OH)-OBu^{t} (3.28 g, 16.2 mmol), DMF (268 ml) and EDPA (2.1 g, 16.2 mmol) was stirred for 64 h at room temperature. The reaction mixture was concentrated *in vacuo* and the residue dissolved in ethyl acetate (50 ml). The resulting solution was washed with 5% aqueous citric acid (2x25 ml). The solvent was concentrated *in vacuo* and the residue dissolved in DMF (36 ml). The resulting solution was added drop wise to a 10% aqueous solution of citric acid (357 ml) and extracted with ethyl acetate (200 ml), and dried (MgSO₄). The solvent was concentrated *in vacuo* to give the crude glutamic acid intermediate. To a mixture of the crude glutamic acid intermediate, N-hydroxysuccinimide (1.85 g, 16.1 mmol) and DMF (25 ml) was added a solution of DCC (3.32 g, 16.1 mmol) in dichloromethane (15 ml). The resulting mixture was stirred at ambient temperature for 20 h. The reaction mixture was filtered and the solvent concentrated *in vacuo.* The residue was purified on a silica gel column (40-63µ), eluted with a mixture of dichloromethane and acetonitril (1:1) to give the title compound (0.63 g, 6% over all).

### Reference Example 6

### Synthesis of desamino-His⁷,Arg²⁶,Lys³⁴ (N^{ε}-(γ-glutamyl(N^{α}-octanoyl))) GLP-1 (7-37)-OH.

To a mixture of desamino-His⁷,Arg²⁶,Lys³⁴ GLP-1 (7-37)-OH (14.3 mg, 4.2 µmol), EDPA (15.3 mg, 119 µmol), NMP (2 ml) and water (1 ml) was added a solution of Cap-Glu(ONSu)-OBu^{t}. (6.8 mg, 12.7 µmol), prepared as described in example 5, in NMP (135 µl). The reaction mixture was gently shaken for 5 min. at room temperature, and then allowed to stand for an additional 2 h at room temperature. The reaction was quenched by the addition of a solution of glycine (7 mg, 93 µmol) in water (698 µl). A 0.5 % aqueous solution of ammonium acetate (42 ml) was added, and the resulting mixture eluted onto a Varian 5g C8 Mega Bond Elut^{®}, the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (4.1 mg, 27 %) was isolated, and the product was analysed by PDMS. The m/z value for the protonated molecular ion was found to be 3626 +- 3. The resulting molecular weight is thus 3625 +- 3 amu (theoretical value 3625 amu).

### Example 7

### Synthesis of Desamino-His⁷,Arg³⁴,Lys²⁶(N^{ε}-(γ-glutamyl(N^{α}-hexadecanoyl))) GLP-1 (7-37)

To a mixture of desamino-His⁷,Arg³⁴-GLP-1 (7-37)-OH (20 mg, 5.9 µmol), EDPA (21.5 mg, 166 µmol), NMP (2.8 ml) and water (1.4 ml) was added a solution Pal-Glu(ONSu)-OBu^{t} (9.6 mg, 17.8 µmol in NMP (240 µl). The reaction mixture was gently shaken for 5 min., and then allowed to stand for an additional 75 min. at room temperature. The reaction was quenched by the addition of a solution of glycine (9.8 mg, 130 µmol) in water (979 µl). A 0.5% aqueous solution of ammonium acetate (58 ml) was added, and the resulting mixture eluted onto a Varian 5g C8 Mega Bond Elut^{®}, the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (9.1 mg, 41%) was isolated, and the product was analysed by PDMS. The m/z value for the protonated molecular ion was found to be 3739 ± 3. The resulting molecular weight is thus 3738 ± 3 AMU (theoretical value 3736 AMU).

### Example 8

### Synthesis of Myr-GABA-ONSu

To a solution of Myr-ONSu (4 g, 12.3 mmol) in DMF (350 ml) was added EDPA (1.58 g, 12.3 mmol) and γ-aminobutyric acid (1.26 g, 12.3 mmol). The resulting mixture was stirred at ambient temperature for 18 h. Water (50 ml) was added and the solution stirred for 1 h at room temperature. The solvents were removed *in vacuo* to give a solid. The solid residue was dissolved in DMF (75 ml) and the solution added drop by drop to a 5% aqueous solution of citric acid (250 ml). The precipitate collected, washed with water (100 ml) and dried *in vacuo* to give the free acid intermediate (3.65 g, 95%). To a solution of the free acid intermediate (3 g, 9.6 mmol), N-hydroxysuccinimide (1.65 g, 14.4 mmol) and N-(3-dimethylaminopropyl)-N'-ethylcarbodiimide hydrochloride (3.67 g, 19.1 mmol) in DMF (330 ml) was stirred for 18 h at room temperature, and the solvent removed *in vacuo* to give a solid. The solid residue was dissolved in dichloromethane (100 ml) and washed with brine (100 ml). The organic phase was dried (MgSO₄) and concentrated *in vacuo* to give a solid. The solid residue was recrystallised from n-heptane (75 ml) to give the title compound (2.8 g, 71%).

### Example 9

### Synthesis of desamino-His⁷,Arg³⁴,Lys²⁶(N^{ε}-(γ-aminobutyroyl(N^{γ}-hexadecanoyl))) GLP-1 (7-37)

To a mixture of desamino-His⁷,Arg³⁴-GLP-1 (7-37)-OH (20 mg, 8.9 µmol), EDPA (21.5 mg, 166 µmol), NMP (2.8 ml) and water (1.4 ml) was added a solution Pal-GABA-ONSu (7.8 mg, 17.8 µmol) in NMP (181 µl). The reaction mixture was gently shaken for 5 min., and then allowed to stand for an additional 90 min. at room temperature. The reaction was quenched by the addition of a solution of glycine (9.3 mg, 125 µmol) in water (93 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (11.6 mg, 55%) was isolated, and the product was analysed by PDMS. The m/z value for the protonated molecular ion was found to be 3692 ± 3. The resulting molecular weight is thus 3691 ± 3 AMU (theoretical value 3693 AMU).

### Example 10

### Synthesis of desamino-His⁷,Arg³⁴,Lys²⁶(N^{ε}-(β-alanyl(N^{γ}-hexadecanoyl))) GLP-1 (7-37)

To a mixture of desamino-His⁷,Arg³⁴-GLP-1 (7-37)-OH (25 mg, 7.4 µmol), EDPA (26.8 mg, 208 µmol), NMP (3.5 ml) and water (1.75 ml) was added a solution Pal-β-Ala-ONSu (9.4 mg, 22.2 µmol) in NMP (236 µl). The reaction mixture was gently shaken for 5 min., and then allowed to stand for an additional 130 min. at room temperature. The reaction was quenched by the addition of a solution of glycine (12.2 mg, 163 µmol) in water (122 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (13.4 mg, 49%) was isolated, and the product was analysed by PDMS. The m/z value for the protonated molecular ion was found to be 3681 ± 3. The resulting molecular weight is thus 3680 ± 3 AMU (theoretical value 3678 AMU).

### Example 11

### Synthesis of Ste-GABA-ONSu

To a solution of Ste-ONSu (3 g, 7.9 mmol) in DMF (270 ml) was added EDPA (1 g, 7.9 mmol) and a solution of γ-aminobutyric acid (0.81 g, 7.9 mmol) in water (40 ml). The resulting suspension was stirred at ambient temperature for 18 h, and then concentrated *in vacuo* to a final volume of 50 ml. The resulting suspension was added to a 5% aqueous solution of citric acid (500 ml) whereby a precipitate is formed. The precipitate was collected and washed with water (50 ml), and dried *in vacuo* for 4h to give the free acid intermediate (2.8 g, 97%). To a mixture of the free acid intermediate (2.6 g, 7 mmol), N-hydroxysuccinimide (1.21 g, 10.5 mmol) and N-(3-dimethylaminopropyl)-N'-ethylcarbodiimide hydrochloride (2.69 g, 14 mmol) in NMP (300 ml) was stirred for 70 h, and the solvent removed *in vacuo* to give a solid. The solid residue was dissolved in dichloromethane (100 ml) and washed with brine (2x100 ml). The organic phase was dried (MgSO₄) and concentrated *in vacuo* to give a solid. The solid residue was recrystallised from n-heptane (75 ml) to give the title compound (2.2 g, 67%).

### Example 12

Synthesis of Arg³⁴,Ala⁸(N^{α}-(imidazol-4-ylprop-2-enoyl),Lys²⁶(N^{ε}-(γ-aminobutyroyl(N^{γ}-hexadecanoyl))) GLP-1 (8-37)

To a mixture of Arg³⁴,Ala⁸(N^{α}-(imidazol-4-ylprop-2-enoyl)-GLP-1 (8-37)-OH (5.6 mg, 1.7 µmol), EDPA (6 mg, 46.2 µmol), NMP (0.78 ml) and water (0.39 ml) was added a solution Pal-GABA-ONSu (2.2 mg, 5 µmol) in NMP (54 µl). The reaction mixture was gently shaken for 5 min., and then allowed to stand for an additional 80 min. at room temperature. The reaction was quenched by the addition of a solution of glycine (2.7 mg, 36 µmol) in water (27 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (1.9 mg, 31 %) was isolated, and the product was analysed by PDMS. The m/z value for the protonated molecular ion was found to be 3690 ± 3. The resulting molecular weight is thus 3689 ± 3 AMU (theoretical value 3690 AMU).

### Example 13

### Synthesis of Arg³⁴,Ala⁸(N^{α}-(imidazol-4-ylacetyl),Lys²⁶(N^{ε}-(γ-aminobutyroyl(N^{γ}-hexadecanoyl))) GLP-1 (8-37)

To a mixture of Arg³⁴,Ala⁸(N^{α}-(imidazol-4-ylacetyl)-GLP-1 (8-37)-OH (5.3 mg, 1.6 µmol), EDPA (5.7 mg, 43.9 µmol), NMP (0.74 ml) and water (0.37 ml) was added a solution Pal-GABA-ONSu (2 mg, 4.7 µmol) in NMP (52 µl). The reaction mixture was gently shaken for 5 min., and then allowed to stand for an additional 80 min. at room temperature. The reaction was quenched by the addition of a solution of glycine (2.6 mg, 34 µmol) in water (26 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (2.2 mg, 38%) was isolated, and the product was analysed by PDMS. The m/z value for the protonated molecular ion was found to be 3676 ± 3. The resulting molecular weight is thus be 3675 ± 3 AMU (theoretical value 3678 AMU).

### Example 14

### Synthesis of desamino-His⁷,Arg³⁴,Lys²⁶(Nε-(γ-aminobutyroyl(N^{γ}-tetradecanoyl))) GLP-1 (7-37)

To a mixture of desamino-His⁷,Arg³⁴-GLP-1 (7-37)-OH (25 mg, 7.4 µmol), EDPA (26.9 mg, 208 µmol), NMP (3.5 ml) and water (1.75 ml) was added a solution Myr-GABA-ONSu (9.1 mg, 22.2 µmol), prepared as described in example 8, in NMP (228 µl). The reaction mixture was gently shaken for 5 min., and then allowed to stand for an additional 90 min. at room temperature. The reaction was quenched by the addition of a solution of glycine (12.2 mg, 163 µmol) in water (122 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (10.5 mg, 39%) was isolated, and the product was analysed by PDMS. The m/z value for the protonated molecular ion was found to be 3667 ± 3. The resulting molecular weight is thus 3664 ± 3 AMU (theoretical value 3662 AMU).

### Example 15

### Synthesis of desamino-His⁷,Arg³⁴,Lys²⁶(N^{ε}-(γ-aminobutyroyl(N^{γ}-octadecanoyl))) GLP-1 (7-37)

To a mixture of desamino-His⁷,Arg³⁴-GLP-1 (7-37)-OH (25 mg, 7.4 µmol), EDPA (26.8 mg, 207 µmol), NMP (3.5 ml) and water (1.75 ml) was added a solution Ste-GABA-ONSu (10.4 mg, 22.2 µmol), prepared as described in example 11, in NMP (259 µl). The reaction mixture was gently shaken for 5 min., and then allowed to stand for an additional 170 min. at room temperature. The reaction was quenched by the addition of a solution of glycine (12.2 mg, 163 µmol) in water (122 µl) and the reaction mixture purified by column chromatography using a cyanopropyl column (Zorbax 300SB-CN) and a standard acetonitril/TFA system. The column was heated to 65°C and the acetonitril gradient was 0-100% in 60 minutes. The title compound (7 mg, 25%) was isolated, and the product was analysed by PDMS. The m/z value for the protonated molecular ion was found to be 3719 ± 3. The resulting molecular weight is thus 3718 ± 3 AMU (theoretical value 3720 AMU).

## Claims

1. A GLP-1 derivative having a protracted profile of action relative to GLP-1 (7-37) and of the formula III: 
7 8 9 10 11 12 13 14 15 16 17
Xaa-Xaa-Xaa-Gly-Xaa-Phe-Thr-Xaa-Asp-Xaa-Xaa-

18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa (III)
 wherein
Xaa at position 7
is selected from the group consisting of: 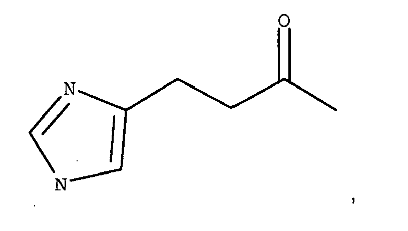 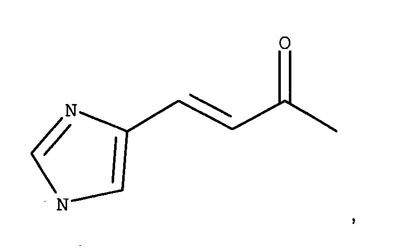 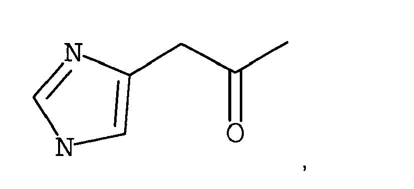 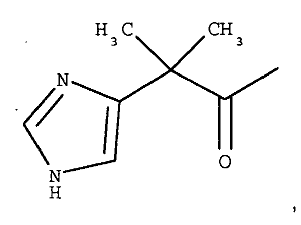 and 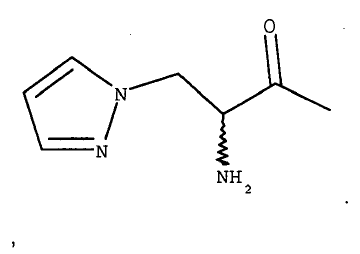 Xaa at position 8 is Ala, Gly, Ser, Thr, or Val,
Xaa at position 9 is Glu,
Xaa at position 11 is Thr,
Xaa at position 14 is Ser,
Xaa at position 16 is Val,
Xaa at position 17 is Ser,
Xaa at position 18 is Ser, Glu, Asp, or Lys,
Xaa at position 19 is Tyr, Glu, Asp, or Lys,
Xaa at position 20 is Leu, Glu, Asp, or Lys,
Xaa at position 21 is Glu, Asp, or Lys,
Xaa at position 22 is Gly, Glu, Asp, or Lys,
Xaa at position 23 is Gln, Glu, Asp, or Lys,
Xaa at position 24 is Ala, Glu, Asp, or Lys,
Xaa at position 25 is Ala, Glu, Asp, or Lys,
Xaa at position 26 is Lys, Arg, Glu, or Asp,
Xaa at position 27 is Glu, Asp, or Lys,
Xaa at position 30 is Ala, Glu, Asp, or Lys,
Xaa at position 31 is Trp, Glu, Asp, or Lys,
Xaa at position 32 is Leu, Glu, Asp, or Lys,
Xaa at position 33 is Val, Glu, Asp, or Lys,
Xaa at position 34 is Lys, Arg, Glu, or Asp,
Xaa at position 35 is Gly, Glu, Asp, or Lys,
Xaa at position 36 is Arg, Lys, Glu, or Asp,
Xaa at position 37 is Gly, Glu, Asp, or Lys,
Xaa at position 38 is deleted,
Xaa at position 39 is deleted,
Xaa at position 40 is deleted,
Xaa at position 41 is deleted,
Xaa at position 42 is deleted,
Xaa at position 43 is deleted,
Xaa at position 44 is deleted, and
Xaa at position 45 is deleted, or
(a) a C-1-6-ester thereof, (b) an amide, C-1-6-alkylamide, or C-1-6-dialkylamide thereof, and/or (c) a pharmaceutically acceptable salt thereof,
wherein
(i) a lipophilic substituent comprising from 12 to 24 carbon atoms is attached via a spacer to one or more of (a) the amino group of the N-terminal amino acid, (b) the carboxy group of the C-terminal amino acid, (c) the ε-amino group of Lys, and/or (d) the carboxy group which is part of the R group of Asp or Glu, and
(ii) the total number of different amino acids of the GLP-1 peptide as compared to the corresponding native form of GLP-1 is one, two, three, four, five, or six.

2. The GLP-1 derivative of claim 1, wherein only one or two Lys are present.

3. The GLP-1 derivative of claim 2, wherein only one Lys is present.

4. The GLP-1 derivative of any of claims 1-3, wherein Lys is at the carboxy-terminus.

5. The GLP-1 derivative of any of claims 1-4, wherein Glu or Asp is adjacent to Lys.

6. The GLP-1 derivative of any of claims 1-5, wherein the total number of different amino acids between the GLP-1 peptide and the corresponding native form of GLP-1 is five.

7. The GLP-1 derivative of any of claims 1-5, wherein the total number of different amino acids between the derivative of the GLP-1 peptide and the corresponding native form of GLP-1 is four.

8. The GLP-1 derivative of any of claims 1-5, wherein the total number of different amino acids between the derivative of the GLP-1 peptide and the corresponding native form of GLP-1 is three.

9. The GLP-1 derivative of any of claims 1-5, wherein the total number of different amino acids between the derivative of the GLP-1 peptide and the corresponding native form of GLP-1 is two.

10. The GLP-1 derivative of any of claims 1-5, wherein the total number of different amino acids between the derivative of the GLP-1 peptide and the corresponding native form of GLP-1 is one.

11. The GLP-1 derivative of claim 1, wherein Xaa at position 26 is Arg, each of Xaa at positions 38-45 is deleted, and each of the other Xaa is the amino acid in native GLP-1 (7-37).

12. The GLP-1 derivative of claim 1, wherein Xaa at position 34 is Arg, each of Xaa at positions 38-45 is deleted, and each of the other Xaa is the amino acid in native GLP-1 (7-37).

13. The GLP-1 derivative of claim 1, wherein Xaa at positions 26 and 34 is Arg, Xaa at position 36 is Lys, each of Xaa at positions 38-45 is deleted, and each of the other Xaa is the amino acid in native GLP-1 (7-37).

14. The GLP-1 derivative of claim 1, wherein Xaa at position 8 is Thr, Ser, Gly or Val, Xaa at position 37 is Glu, Xaa at position 36 is Lys, each of Xaa at positions 38-45 is deleted, and each of the other Xaa is the amino acid in native GLP-1 (7-37).

15. The GLP-1 derivative of claim 1, wherein Xaa at position 18, 23 or 27 is Lys, and Xaa at positions 26 and 34 is Arg, each of Xaa at positions 38-45 is deleted, and each of the other Xaa is the amino acid in native GLP-1(7-37).

16. The GLP-1 derivative of claim 1, wherein Xaa at position 8 is Thr, Ser, Gly, or Val, Xaa at position 18, 23 or 27 is Lys, and Xaa at position 26 and 34 is Arg, each of Xaa at positions 38-45 is deleted, and each of the other Xaa is the amino acid in native GLP-1 (7-37).

17. The GLP-1 derivative of any of claims 1-16 wherein three lipophilic substituents are present.

18. The GLP-1 derivative of any of claims 1-16 wherein two lipophilic substituents are present.

19. The GLP-1 derivative of any of claims 1-16 wherein one lipophilic substituent is present.

20. The GLP-1 derivative of any of claims 1-19, wherein a lipophilic substituent is attached to the amino group of the N-terminal amino acid residue of the GLP-1 peptide.

21. The GLP-1 derivative of any of claims 1-20, wherein a lipophilic substituent is attached to the carboxy group of the C-terminal amino acid residue of the GLP-1 peptide.

22. The GLP-1 derivative of any of claims 1-21, wherein a lipophilic substituent is attached to the carboxy group which is part of the R group of Asp or Glu of the GLP-1 peptide.

23. The GLP-1 derivative of any of claims 1-22, wherein a lipophilic substituent is attached to an ε-amino group of Lys of the GLP-1 peptide.

24. The GLP-1 derivative of any of claims 1-24, wherein a lipophilic substituent is attached to an amino acid residue in such a way that a carboxyl group of the lipophilic substituent forms an amide bond with the ε-amino group of Lys of the GLP-1 peptide.

25. The GLP-1 derivative of claim 24, wherein the spacer is an unbranched alkane α,ω-dicarboxylic acid group having from 1 to 7 methylene groups, preferably two methylene groups, which forms an amide bond with an amino group of the parent GLP-1 peptide and an amide bond with an amino group of the lipophilic substituent.

26. The GLP-1 derivative of claim 24, wherein the spacer is an amino acid residue except Cys or Met, or a dipeptide such as Gly-Lys.

27. The GLP-1 derivative of claim 26, wherein the ε-amino group of Lys forms an amide bond with a carboxylic group of the amino acid residue or dipeptide spacer, and an amino group of the amino acid residue or dipeptide spacer forms an amide bond with a carboxyl group of the lipophilic substituent.

28. The GLP-1 derivative of any of claims 24-27, wherein the spacer is γ-L-glutamyl, β-L-asparagyl, β-alanyl, glycyl, or α-(γ-aminobutanoyl).

29. The GLP-1 derivative of any of claims 1-28, wherein the lipophilic substituent comprises a partially or completely hydrogenated cyclopentanophenanthrene skeleton.

30. The GLP-1 derivative of any of claims 1-28, wherein the lipophilic substituent is a straight-chain or branched alkyl group.

31. The GLP-1 derivative of any of claims 1-28 wherein the lipophilic substituent is an acyl group of a straight-chain or branched fatty acid, preferably an acyl group of a straight-chain fatty acid.

32. The GLP-1 derivative of claim 32 wherein the acyl group is selected from CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

33. The GLP-1 derivative of claim 32 wherein the acyl group is tetradecanoyl.

34. The GLP-1 derivative of claim 32 wherein the acyl group is hexadecanoyl.

35. The GLP-1 derivative of claim 32 wherein the acyl group is octadecanoyl.

36. The GLP-1 derivative of any of the preceding claims, which is selected from
desamino-His⁷,Arg²⁶,Lys³⁴ (N^{ε}-(γ-glutamyl(N^{α}-hexadecanoyl))) GLP-1 (7-37)-OH,
desamino-His⁷,Arg³⁴, Lys²⁶(N^{ε}-(y-glutamyl(N^{α}-hexadecanoyl))) GLP-1 (7-37),
desamino-His⁷,Arg³⁴, Lys²⁶(N^{ε}-(γ-aminobutyroyl(N^{γ}-hexadecanoyl))) GLP-1 (7-37),
desamino-His⁷,Arg³⁴, Lys²⁶(N^{ε}-(β-alanyl(N^{γ}-hexadecanoyl))) GLP-1 (7-37),
Arg³⁴,Ala⁸(N^{α}-(imidazol-4-ylprop-2-enoyl),Lys²⁶(N^{ε}-(γ-aminobutyroyl(N^{γ}-hexadecanoyl))) GLP-1 (8-37),
Arg³⁴,Ala⁸(N^{α}-(imidazol-4-ylacetyl),Lys²⁶(N^{ε}-(γ-aminobutyroyl(N^{γ}-hexadecanoyl))) GLP-1 (8-37),
desamino-His⁷,Arg³⁴, Lys²⁶(N^{ε}-(γ-aminobutyroyl(N^{γ}-tetradecanoyl))) GLP-1 (7-37), and
desamino-His⁷,Arg³⁴, Lys²⁶(N^{ε}-(γ-aminobutyroyl(N^{γ}-octadecanoyl))) GLP-1 (7-37).

37. The GLP-1 derivative of any of claims 1-30 wherein the lipophilic substituent is an acyl group of a straight-chain or branched alkane α,ω-dicarboxylic acid.

38. The GLP-1 derivative of claim 37 wherein the acyl group is selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

39. The GLP-1 derivative of any of claims 1-20, wherein the lipophilic substituent with the attached spacer is a group of the formula CH₃(CH₂)ₚNH-CO(CH₂)₂CO-, wherein p is an integer of from 8 to 33, preferably from 12 to 28.

40. The GLP-1 derivative of any of claims 1-20, wherein the lipophilic substituent with the attached spacer is a group of the formula CH₃(CH₂)ᵣCO-NHCH(COOH)(CH₂)₂CO-, wherein r is an integer of from 10 to 24.

41. The GLP-1 derivative of any of claims 1-20, wherein the lipophilic substituent with the attached spacer is a group of the formula CH₃(CH₂)ₛCO-NHCH((CH₂)₂COOH)CO-, wherein s is an integer of from 8 to 24.

42. The GLP-1 derivative of any of claims 1-20, wherein the lipophilic substituent with the attached spacer is a group of the formula -NHCH(COOH)(CH₂)₄NH-CO(CH₂)ᵤCH₃, wherein u is an integer of from 8 to 18.

43. The GLP-1 derivative of any of claims 1-20, wherein the lipophilic substituent with the attached spacer is a group of the formula -NHCH(COOH)(CH₂)₄NH-COCH((CH₂)₂COOH)NH-CO(CH₂)_{w}CH₃, wherein w is an integer of from 10 to 16.

44. The GLP-1 derivative of any of claims 1-20, wherein the lipophilic substituent with the attached spacer is a group of the formula -NHCH(COOH)(CH₂)₄NH-CO(CH₂)₂CH(COOH)NH-CO(CH₂)ₓCH₃, wherein x is an integer of from 10 to 16.

45. The GLP-1 derivative of any of claims 1-20, wherein the lipophilic substituent with the attached spacer is a group of the formula -NHCH(COOH)(CH₂)₄NH-CO(CH₂)₂CH(COOH)NH-CO(CH₂)_{y}CH₃, wherein y is zero or an integer of from 1 to 22.

46. The GLP-1 derivative of any of claims 1-45 which has insulinotropic activity, ability to decrease glucagon, ability to suppress gastric motility, ability to restore glucose competency to beta-cells, and/or ability to suppress appetite/reduce weight.

47. A pharmaceutical composition comprising a GLP-1 derivative of any of claims 1-46 and a pharmaceutically acceptable vehicle or carrier.

48. The pharmaceutical composition of claim 47, further comprising another antidiabetic agent.

49. The pharmaceutical composition of claim 48, wherein the antidiabetic agent is an insulin, more preferably human insulin.

50. The pharmaceutical composition of claim 48, wherein the antidiabetic agent is a hypoglycaemic agent.

51. The pharmaceutical composition of claim 47, further comprising another antiobesity agent.

52. The pharmaceutical composition of claim 51, wherein the antiobesity agent is selected from the group consisting of leptin, amphetamin, dexfenfluramine, sibutramine, orlistat, CART agonists, NPY antagonists, orexin antagonists, H3-antagonists, TNF agonists, CRF agonists, CRF BP antagonists, urocortin agonists, β3 agonists, MSH agonists, CCK agonists, serotonin re-uptake inhibitors, mixed serotonin and noradrenergic compounds, 5HT agonists, bombesin agonists, galanin antagonists, growth hormone, growth hormone releasing compounds, glucagon, TRH agonists, uncoupling protein 2 or 3 modulators, leptin agonists, DA agonists (Bromocriptin, Doprexin), lipase/amylase inhibitors, PPAR modulators, PXR modulators and TR β agonists.

53. Use of a GLP-1 derivative of any of claims 1-46 for the preparation of a medicament with a protracted profile of action relative to GLP-1 (7-37), for the treatment of non-insulin dependent diabetes mellitus.

54. Use of a GLP-1 derivative of any of claims 1-46 for the preparation of a medicament with a protracted profile of action relative to GLP-1 (7-37), for the treatment of insulin dependent diabetes mellitus.

55. Use of a GLP-1 derivative of any of claims 1-46 for the preparation of a medicament with a protracted profile of action relative to GLP-1 (7-37), for the treatment of obesity.

56. A GLP-1 derivative according to any one of claims 1-46 for use as a medicament.

57. A GLP-1 derivative according to any one of claims 1-46 for the preparation of a medicament for the treatment of insulin dependent or non-insulin dependent diabetes mellitus.

58. A GLP-1 derivative according to any one of claims 1-46 for the preparation of a medicament for the treatment of obesity.

59. A GLP-1 derivative according to any one of claims 1-46 for the preparation of a medicament for the treatment of insulin resistance.

60. A GLP-1 derivative according to any one of claims 1-46 for use in the treatment of insulin dependent or non-insulin dependent diabetes mellitus.

61. A GLP-1 derivative according to any one of claims 1-46 for use in the treatment of obesity.

62. A GLP-1 derivative according to any one of claims 1-46 for use in treatment of insulin resistance.

## Patentansprüche

1. GLP-1-Derivat mit einem verzögerten Wirkungsprofil in Bezug auf GLP-1(7-37) und der Formel III: 
7   8   9  10  11  12  13  14  15  16  17
Xaa-Xaa-Xaa-Gly-Xaa-Phe-Thr-Xaa-Asp-Xaa-Xaa-

18  19  20  21  22  23  24  25  26  27  28
Xaa-Xaa-Xaa-Xaa-Xaa-Xaa-Xaa-Xaa-Xaa-Xaa-Phe-

29  30  31  32  33  34  35  36  37  38
Ile-Xaa-Xaa-Xaa-Xaa-Xaa-Xaa-Xaa-Xaa-Xaa

39  40  41  42  43  44  45
Xaa-Xaa-Xaa-Xaa-Xaa-Xaa-Xaa                    (III)
 wobei
Xaa an Position 7
ausgewählt ist aus der Gruppe, bestehend aus: 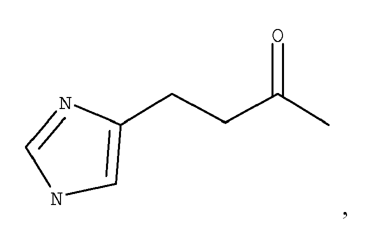 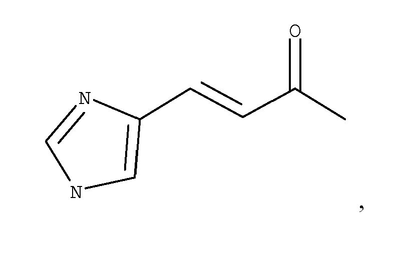 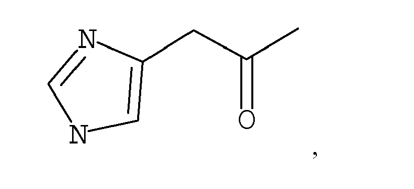 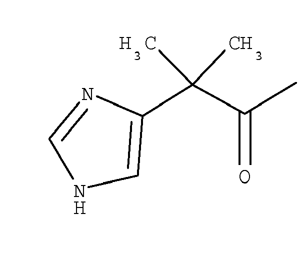 und 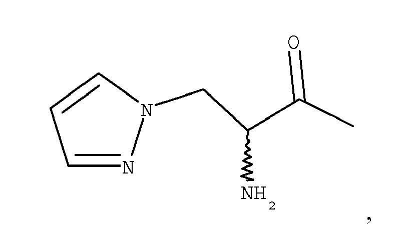 Xaa an Position 8 für Ala, Gly, Ser, Thr oder Val steht,
Xaa an Position 9 für Glu steht,
Xaa an Position 11 für Thr steht,
Xaa an Position 14 für Ser steht,
Xaa an Position 16 für Val steht,
Xaa an Position 17 für Ser steht,
Xaa an Position 18 für Ser, Glu, Asp oder Lys steht,
Xaa an Position 19 für Tyr, Glu, Asp oder Lys steht,
Xaa an Position 20 für Leu, Glu, Asp oder Lys steht,
Xaa an Position 21 für Glu, Asp oder Lys steht,
Xaa an Position 22 für Gly, Glu, Asp oder Lys steht,
Xaa an Position 23 für Gln, Glu, Asp oder Lys steht,
Xaa an Position 24 für Ala, Glu, Asp oder Lys steht,
Xaa an Position 25 für Ala, Glu, Asp oder Lys steht,
Xaa an Position 26 für Lys, Arg, Glu oder Asp steht,
Xaa an Position 27 für Glu, Asp oder Lys steht,
Xaa an Position 30 für Ala, Glu, Asp oder Lys steht,
Xaa an Position 31 für Trp, Glu, Asp oder Lys steht,
Xaa an Position 32 für Leu, Glu, Asp oder Lys steht,
Xaa an Position 33 für Val, Glu, Asp oder Lys steht,
Xaa an Position 34 für Lys, Arg, Glu oder Asp steht,
Xaa an Position 35 für Gly, Glu, Asp oder Lys steht,
Xaa an Position 36 für Arg, Lys, Glu oder Asp steht,
Xaa an Position 37 für Gly, Glu, Asp oder Lys steht,
Xaa an Position 38 entfernt ist,
Xaa an Position 39 entfernt ist,
Xaa an Position 40 entfernt ist,
Xaa an Position 41 entfernt ist,
Xaa an Position 42 entfernt ist,
Xaa an Position 43 entfernt ist,
Xaa an Position 44 entfernt ist und
Xaa an Position 45 entfernt ist oder
(a) ein C-1-6-Ester davon, (b) ein Amid, C-1-6-Alkylamid oder C-1-6-Dialkylamid davon, und/oder (c) ein pharmazeutisch verträgliches Salz davon,
wobei
(i) ein lipophiler Substituent, umfassend 12 bis 24 Kohlenstoffatome, über einen Abstandhalter an eine oder mehrere (a) der Aminogruppe, der N-terminalen Aminosäure, (b) der Carboxygruppe der C-terminalen Aminosäure, (c) der ε-Aminogruppe von Lys und/oder (d) der Carboxygruppe, die Teil der R-Gruppe von Asp oder Glu ist angelagert ist und
(ii) die Gesamtanzahl an verschiedenen Aminosäuren des GLP-1-Peptids verglichen mit der entsprechenden nativen Form von GLP-1 eins, zwei, drei, vier, fünf oder sechs beträgt.

2. GLP-1-Derivat nach Anspruch 1, wobei nur ein oder zwei Lys vorliegen.

3. GLP-1-Derivat nach Anspruch 2, wobei nur ein Lys vorliegt.

4. GLP-1-Derivat nach einem der Ansprüche 1-3, wobei Lys am Carboxy-Terminus vorliegt.

5. GLP-1-Derivat nach einem der Ansprüche 1-4, wobei Glu oder Asp neben Lys vorliegen.

6. GLP-1-Derivat nach einem der Ansprüche 1-5, wobei die Gesamtanzahl von verschiedenen Aminosäuren zwischen dem GLP-1-Peptid und der entsprechenden nativen Form von GLP-1 fünf beträgt.

7. GLP-1-Derivat nach einem der Ansprüche 1-5, wobei die Gesamtanzahl von verschiedenen Aminosäuren zwischen dem Derivat des GLP-1-Peptids und der entsprechenden nativen Form von GLP-1 vier beträgt.

8. GLP-1-Derivat nach einem der Ansprüche 1-5, wobei die Gesamtanzahl von verschiedenen Aminosäuren zwischen dem Derivat des GLP-1-Peptids und der entsprechenden nativen Form von GLP-1 drei beträgt.

9. GLP-1-Derivat nach einem der Ansprüche 1-5, wobei die Gesamtanzahl von verschiedenen Aminosäuren zwischen dem Derivat des GLP-1-Peptids und der entsprechenden nativen Form von GLP-1 zwei beträgt.

10. GLP-1-Derivat nach einem der Ansprüche 1-5, wobei die Gesamtanzahl von verschiedenen Aminosäuren zwischen dem Derivat des GLP-1-Peptids und der entsprechenden nativen Form von GLP-1 eins beträgt.

11. GLP-1-Derivat nach Anspruch 1, wobei Xaa an Position 26 für Arg steht, jedes von Xaa an den Positionen 38-45 entfernt ist und jedes der anderen Xaa für die Aminosäure in nativem GLP-1(7-37) steht.

12. GLP-1-Derivat nach Anspruch 1, wobei Xaa an Position 34 für Arg steht, jedes von Xaa an den Positionen 38-45 entfernt ist und jedes der anderen Xaa für die Aminosäure in nativem GLP-1(7-37) steht.

13. GLP-1-Derivat nach Anspruch 1, wobei Xaa an den Positionen 26 und 3 4 für Arg steht, Xaa an Position 36 für Lys steht, jedes von Xaa an den Positionen 38-45 entfernt ist und jedes der anderen Xaa für die Aminosäure in nativem GLP-1 (7-37) steht.

14. GLP-1-Derivat nach Anspruch 1, wobei Xaa an Position 8 für Thr, Ser, Gly oder Val steht, Xaa an Position 37 für Glu steht, Xaa an Position 36 für Lys steht, jedes von Xaa an den Positionen 38-45 entfernt ist und jedes der anderen Xaa für die Aminosäure in nativem GLP-1(7-37) steht.

15. GLP-1-Derivat nach Anspruch 1, wobei Xaa an Position 18, 23 oder 27 für Lys steht und Xaa an den Positionen 26 und 34 für Arg steht, jedes von Xaa an den Positionen 38-45 entfernt ist und jedes der anderen Xaa für die Aminosäure in nativem GLP-1(7-37) steht.

16. GLP-1-Derivat nach Anspruch 1, wobei Xaa an Position 8 für Thr, Ser, Gly oder Val steht, Xaa an Position 18, 23 oder 27 für Lys steht und Xaa an Position 26 und 34 für Arg steht, jedes von Xaa an den Positionen 38-45 entfernt ist und jedes der anderen Xaa für die Aminosäure in nativem GLP-1(7-37) steht.

17. GLP-1-Derivat nach einem der Ansprüche 1-16, wobei drei lipophile Substituenten vorliegen.

18. GLP-1-Derivat nach einem der Ansprüche 1-16, wobei zwei lipophile Substituenten vorliegen.

19. GLP-1-Derivat nach einem der Ansprüche 1-16, wobei ein lipophiler Substituent vorliegt.

20. GLP-1-Derivat nach einem der Ansprüche 1-19, wobei ein lipophiler Substituent an die Aminogruppe des N-terminalen Aminosäurerests des GLP-1-Peptids angelagert ist.

21. GLP-1-Derivat nach einem der Ansprüche 1-20, wobei ein lipophiler Substituent an die Carboxygruppe des C-terminalen Aminosäurerests des GLP-1-Peptids angelagert ist.

22. GLP-1-Derivat nach einem der Ansprüche 1-21, wobei ein lipophiler Substituent an die Carboxygruppe angelagert ist, die Teil der R-Gruppe von Asp oder Glu des GLP-1-Peptids ist.

23. GLP-1-Derivat nach einem der Ansprüche 1-22, wobei ein lipophiler Substituent an eine ε-Aminogruppe von Lys des GLP-1-Peptids angelagert ist.

24. GLP-1-Derivat nach einem der Ansprüche 1-24, wobei ein lipophiler Substituent in einer derartigen Weise an einen Aminosäurerest angelagert ist, dass eine Carboxygruppe des lipophilen Substituenten mit der ε-Aminogruppe von Lys des GLP-1-Peptids eine Amidbindung bildet.

25. GLP-1-Derivat nach Anspruch 24, wobei es sich bei dem Abstandhalter um eine unverzweigte Alkan-α,ω-dicarbonsäuregruppe mit 1 bis 7 Methylengruppen, vorzugsweise zwei Methylengruppen handelt, die mit einer Aminogruppe des parentalen GLP-1-Peptids eine Amidbindung und mit einer Aminogruppe des lipophilen Substituenten eine Amidbindung bildet.

26. GLP-1-Derivat nach Anspruch 24, wobei es sich bei dem Abstandhalteer um einen Aminosäurerest mit Ausnahme von Cys oder Met oder ein Dipeptid wie Gly-Lys handelt.

27. GLP-1-Derivat nach Anspruch 26, wobei die ε-Aminogruppe von Lys mit einer Carbonsäuregruppe des Aminosäurerest- oder Dipeptidabstandhalters eine Amidbindung bildet und eine Aminogruppe des Aminosäurerest- oder Dipeptidabstandhalters mit einer Carboxygruppe des lipophilen Substituenten eine Amidbindung bildet.

28. GLP-1-Derivat nach einem der Ansprüche 24-27, wobei es sich bei dem Abstandhalter um γ-L-Glutamyl, β-L-Asparagyl, β-Alanyl, Glycyl oder α-(γ-Aminobutanoyl) handelt.

29. GLP-1-Derivat nach einem der Ansprüche 1-28, wobei der lipophile Substituent ein teilweise oder vollständig hydriertes Cyclopentanphenanthrengerüst umfasst.

30. GLP-1-Derivat nach einem der Ansprüche 1-28, wobei es sich bei dem lipophilen Substituenten um eine geradkettige oder verzweigte Alkylgruppe handelt.

31. GLP-1-Derivat nach einem der Ansprüche 1-28, wobei es sich bei dem lipophilen Substituenten um eine Acylgruppe einer geradkettigen oder verzweigten Fettsäure, vorzugsweise eine Acylgruppe einer geradkettigen Fettsäure handelt.

32. GLP-1-Derivat nach Anspruch 32, wobei die Acylgruppe ausgewählt ist aus CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- und CH₃(CH₂)₂₂CO-.

33. GLP-1-Derivat nach Anspruch 32, wobei es sich bei der Acylgruppe um Tetradecanoyl handelt

34. GLP-1-Derivat nach Anspruch 32, wobei es sich bei der Acylgruppe um Hexadecanoyl handelt.

35. GLP-1-Derivat nach Anspruch 32, wobei es sich bei der Acylgruppe um Octadecanoyl handelt.

36. GLP-1-Derivat nach einem der vorangehenden Ansprüche, das ausgewählt ist aus
Desamino- His⁷,Arg²⁶,Lys³⁴ (N^{ε}-(γ-glutamylol(N^{α}-hexadecanoyl)))-GLP-1(7-37)-OH,
Desamino-His⁷,Arg³⁴,Lys²⁶(N^{ε}-(γ-glutamyl(N^{α}-hexadecanoyl)))-GLP-1(7-37),
Desamino-His⁷,Arg³⁴,Lys²⁶(N^{ε}-(γ-aminobutyroyl(N^{γ}-hexadecanoyl)))-GLP-1(7-37),
Desamino-His⁷,Arg³⁴,Lys²⁶(N^{ε}-(β-alanyl(N^{γ}-hexadecanoyl)))-GLP-1(7-37),
Arg³⁴,Ala⁸(N^{α}-(imidazol-4-ylprop-2-enoyl),Lys²⁶(N^{ε}-(γ-aminobutyroyl(N^{γ}-hexadecanoyl)))-GLP-1(8-37),
Arg³⁴,Ala⁸(N^{α}-(imidazol-4-ylacetyl),Lys²⁶(N^{ε}-(γ-aminobutyroyl(N^{γ}-hexadecanoyl)))-GLP-1 (8-3 7), Desamino-His⁷,Arg³⁴,Lys²⁶(N^{ε}-(γ-aminobutyroyl(N^{γ}-tetradecanoyl)))-GLP-1(7-37) und Desamino-His⁷,Arg³⁴,Lys²⁶(NE-(γ-aminobutyroyl(N^{γ}-octadecanoyl)))-GLP-1(7-37).

37. GLP-1-Derivat nach einem der Ansprüche 1-30, wobei es sich bei dem lipophilen Substituenten um eine geradkettige oder verzweigte Alkan-α,ω-dicarbonsäure handelt.

38. GLP-1-Derivat nach Anspruch 37, wobei die Acylgruppe ausgewählt ist aus der Gruppe, umfassend HOOC(CH₂)₁₄CO-, HOOC(CH₂)₁₆CO-, HOOC(CH₂)₁₈CO-, HOOC(CH₂)₂₀CO- und HOOC(CH₂)₂₂CO-.

39. GLP-1-Derivat nach einem der Ansprüche 1-20, wobei es sich bei dem lipophilen Substituenten mit dem Abstandhalter um eine Gruppe der Formel CH₃(CH₂)pNH-CO(CH₂)₂CO- handelt, wobei p für eine ganze Zahl von 8 bis 33, vorzugsweise von 12 bis 28 steht.

40. GLP-1-Derivat nach einem der Ansprüche 1-20, wobei es sich bei dem lipophilen Substituenten mit dem Abstandhalter um eine Gruppe der Formel CH₃(CH₂)ᵣCO-NHCH(COOH)(CH₂)₂CO- handelt, wobei r für eine ganze Zahl von 10 bis 24 steht.

41. GLP-1-Derivat nach einem der Ansprüche 1-20, wobei es sich bei dem lipophilen Substituenten mit dem Abstandhalter um eine Gruppe der Formel CH₃(CH₂)ₛCO-NHCH((CH₂)₂COOH)CO- handelt, wobei s für eine ganze Zahl von 8 bis 24 steht.

42. GLP-1-Derivat nach einem der Ansprüche 1-20, wobei es sich bei dem lipophilen Substituenten mit dem Abstandhalter um eine Gruppe der Formel -NHCH(COOH)(CH₂)₄NH-CO(CH₂)ᵤCH₃ handelt, wobei u für eine ganze Zahl von 8 bis 18 steht.

43. GLP-1-Derivat nach einem der Ansprüche 1-20, wobei es sich bei dem lipophilen Substituenten mit dem Abstandhalter um eine Gruppe der Formel -NHCH(COOH)(CH₂)₄NH-COCH((CH₂)₂COOH)NH-CO(CH₂)_{w}CH₃ handelt, wobei w für eine ganze Zahl von 10 bis 16 steht.

44. GLP-1-Derivat nach einem der Ansprüche 1-20, wobei es sich bei dem lipophilen Substituenten mit dem Abstandhalter um eine Gruppe der Formel -NHCH(COOH)(CH₂)₄NH-CO(CH₂)₂CH(COOH)NH-CO(CH₂)ₓCH₃ handelt, wobei x für eine ganze Zahl von 10 bis 16 steht.

45. GLP-1-Derivat nach einem der Ansprüche 1-20, wobei es sich bei dem lipophilen Substituenten mit dem Abstandhalter um eine Gruppe der Formel -NHCH(COOH)(CH₂)₄NH-CO(CH₂)₂CH(COOH)NH-CO(CH₂)_{y}CH₃ handelt, wobei y für Null oder eine ganze Zahl von 1 bis 22 steht.

46. GLP-1-Derivat nach einem der Ansprüche 1-45, das eine insulinotrope Aktivität, eine Fähigkeit zur Glucagonverminderung, eine Fähigkeit Magenbeweglichkeitsunterdrückung, eine Fähigkeit zur Glucosekompetenzwiederherstellung von Betazellen und/oder eine Fähigkeit zur Appetitsunterdrückung/Gewichtsreduktion aufweist.

47. Arzneimittel, umfassend ein GLP-1-Derivat nach einem der Ansprüche 1-46 und ein pharmazeutisch verträgliches Vehikulum oder einen pharmazeutisch verträglichen Träger.

48. Arzneimittel nach Anspruch 47, des Weiteren umfassend ein weiteres Antidiabetikum.

49. Arzneimittel nach Anspruch 48, wobei es sich bei dem Antidiabetikum um Insulin, stärker bevorzugt Humaninsulin handelt.

50. Arzneimittel nach Anspruch 48, wobei es sich bei dem Antidiabetikum um ein hypoglykämisches Mittel handelt.

51. Arzneimittel nach Anspruch 47, des Weiteren umfassend ein weiteres Mittel gegen Fettsucht.

52. Arzneimittel nach Anspruch 51, wobei das Mittel gegen Fettsucht ausgewählt ist aus der Gruppe, bestehend aus Leptin, Amphetamin, Dexfenfluramin, Sibutramin Orlistat, CART- Agonisten, NPY-Antagonisten Orexinantagonists, H3-Antagonisten, TNF-Agonisten, CRF-Agonisten, CRF-BP-Antagonisten, Urocortin-Agonisten, β3-Agonisten, MSH-Agonisten, CCK-Agonisten, Serotonin-Wiederaufnahme-Hemmern, gemischten Serotonin- und noradrenergen Verbindungen, 5HT-Agonisten, Bombesin-Agonisten, Galanin-Antagonisten, Wachstumshormon, Wachstumshormon freisetzenden Verbindungen, Glucagon, TRH-Agonisten, Modulatoren des Uncoupling Proteins 2 oder 3, Leptin-Agonisten, DA-Agonisten (Bromocriptin, Doprexin), Lipase/Amylase-Hemmern, PPAR-Modulatoren, PXR-Modulatoren und TR-β-Agonisten.

53. Verwendung eines GLP-1-Derivats nach einem der Ansprüche 1-46 zur Herstellung eines Medikaments mit einem verzögerten Wirkungsprofil in Bezug auf GLP-1(7-37) zur Behandlung von nicht-insulinabhängiger Diabetes mellitus.

54. Verwendung eines GLP-1-Derivats nach einem der Ansprüche 1-46 zur Herstellung eines Medikaments mit einem verzögerten Wirkungsprofil in Bezug auf GLP-1(7-37) zur Behandlung von insulinabhängiger Diabetes mellitus.

55. Verwendung eines GLP-1-Derivats nach einem der Ansprüche 1-46 zur Herstellung eines Medikaments mit einem verzögerten Wirkungsprofil in Bezug auf GLP-1(7-37) zur Behandlung von Fettsucht.

56. GLP-1-Derivat nach einem der Ansprüche 1-46 zur Verwendung als Medikament.

57. GLP-1-Derivat nach einem der Ansprüche 1-46 zur Herstellung eines Medikaments zur Behandlung von insulinabhängiger oder nicht-insulinabhängiger Diabetes mellitus.

58. GLP-1-Derivat nach einem der Ansprüche 1-46 zur Herstellung eines Medikaments zur Behandlung von Fettsucht.

59. GLP-1-Derivat nach einem der Ansprüche 1-46 zur Herstellung eines Medikaments zur Behandlung von Insulinresistenz.

60. GLP-1-Derivat nach einem der Ansprüche 1-46 zur Verwendung bei der Behandlung von insulinabhängiger oder nicht-insulinabhängiger Diabetes mellitus.

61. GLP-1-Derivat nach einem der Ansprüche 1-46 zur Verwendung bei der Behandlung von Fettsucht.

62. GLP-1-Derivat nach einem der Ansprüche 1-46 zur Verwendung bei der Behandlung von Insulinresistenz.

## Revendications

1. Dérivé du GLP-1 ayant un profil d'action prolongé par rapport à celui du GLP-1(7-37) et ayant la formule III : 
7 8 9 10 11 12 13 14 15 16 17
Xaa-Xaa-Xaa-Gly-Xaa-Phe-Thr-Xaa-Asp-Xaa-Xaa-

18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa (III)
 dans laquelle
Xaa en position 7 est choisi dans le groupe consistant en : 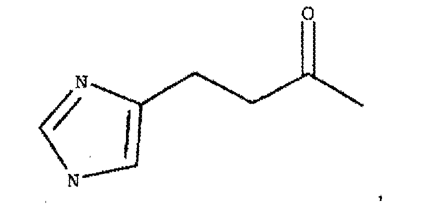 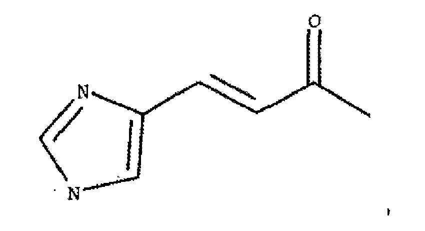 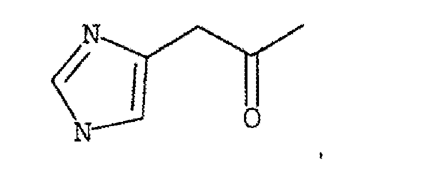 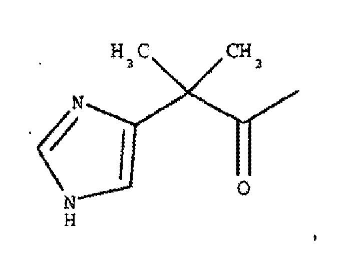 et 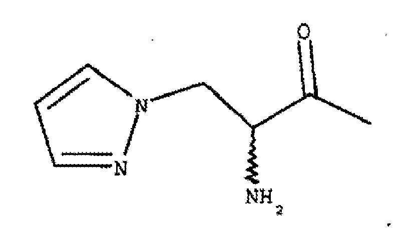 Xaa en position 8 est Ala, Gly, Ser, Thr ou Val,
Xaa en position 9 est Glu,
Xaa en position 11 est Thr,
Xaa en position 14 est Ser,
Xaa en position 16 est Val,
Xaa en position 17 est Ser,
Xaa en position 18 est Ser, Glu, Asp ou Lys,
Xaa en position 19 est Tyr, Glu, Asp ou Lys,
Xaa en position 20 est Leu, Glu, Asp ou Lys,
Xaa en position 21 est Glu, Asp ou Lys,
Xaa en position 22 est Gly, Glu, Asp ou Lys,
Xaa en position 23 est Gln, Glu, Asp ou Lys,
Xaa en position 24 est Ala, Glu, Asp ou Lys,
Xaa en position 25 est Ala, Glu, Asp ou Lys,
Xaa en position 26 est Lys, Arg, Glu ou Asp,
Xaa en position 27 est Glu, Asp ou Lys,
Xaa en position 30 est Ala, Glu, Asp ou Lys,
Xaa en position 31 est Trp, Glu, Asp ou Lys,
Xaa en position 32 est Leu, Glu, Asp ou Lys,
Xaa en position 33 est Val, Glu, Asp ou Lys,
Xaa en position 34 est Lys, Arg, Glu ou Asp,
Xaa en position 35 est Gly, Glu, Asp ou Lys,
Xaa en position 36 est Arg, Lys, Glu ou Asp,
Xaa en position 37 est Gly, Glu, Asp ou Lys,
Xaa en position 38 est délété,
Xaa en position 39 est délété,
Xaa en position 40 est délété,
Xaa en position 41 est délété,
Xaa en position 42 est délété,
Xaa en position 43 est délété,
Xaa en position 44 est délété, et
Xaa en position 45 est délété, ou
(a) l'un de ses esters en C₁₋₆,
(b) l'un de ses amides, alkylamides en C₁₋₆ ou di (alkyle en C₁₋₆)amide, et/ou
(c) l'un de ses acceptables d'un point de vue pharmaceutique,
dans lequel
(i) un substituant lipophile comprenant de 12 à 24 atomes de carbone est fixé par l'intermédiaire d'un espaceur à un ou plusieurs de (a) le groupe amino de l'acide aminé N-terminal, (b) le groupe carboxy de l'acide aminé C-terminal, (c) le groupe ε-amino de Lys et/ou (d) le groupe carboxy qui fait partie du groupe R de sp ou Glu, et
(ii) le nombre total d'acides aminés différents du peptide GLP-1, par comparaison avec la forme native correspondante du GLP-1, est de un, deux, trois, quatre, cinq ou six.

2. Dérivé du GLP-1 selon la revendication 1, dans lequel sont présents seulement un ou deux Lys.

3. Dérivé du GLP-1 selon la revendication 2, dans lequel un seul Lys est présent.

4. Dérivé du GLP-1 selon l'une quelconque des revendications 1 à 3, dans lequel Lys se trouve sur le site carboxy-terminal.

5. Dérivé du GLP-1 selon l'une quelconque des revendications 1 à 4, dans lequel Glu ou Asp est adjacent à Lys.

6. Dérivé du GLP-1 selon l'une quelconque des revendications 1 à 5, dans lequel le nombre total d'acides aminés différents entre le peptide GLP-1 et la forme native correspondante du GLP-1 est de cinq.

7. Dérivé du GLP-1 selon l'une quelconque des revendications 1 à 5, dans lequel le nombre total d'acides aminés différents entre le dérivé du peptide GLP-1 et la forme native correspondante du GLP-1 est de quatre.

8. Dérivé du GLP-1 selon l'une quelconque des revendications 1 à 5, dans lequel le nombre total d'acides aminés différents entre le dérivé du peptide GLP-1 et la forme native correspondante du GLP-1 est de trois.

9. Dérivé du GLP-1 selon l'une quelconque des revendications 1 à 5, dans lequel le nombre total d'acides aminés différents entre le dérivé du peptide GLP-1 et la forme native correspondante du GLP-1 est de deux.

10. Dérivé du GLP-1 selon l'une quelconque des revendications 1 à 5, dans lequel le nombre total d'acides aminés différents entre le dérivé du peptide GLP-1 et la forme native correspondante du GLP-1 est de un.

11. Dérivé du GLP-1 selon la revendication 1, dans lequel Xaa en position 26 est Arg, chacun des Xaa sur les positions 38-45 est délété, et chacun des autres Xaa est l'acide aminé se trouvant dans le GLP-1(7-37) natif.

12. Dérivé du GLP-1 selon la revendication 1, dans lequel Xaa en position 34 est Arg, chacun des Xaa sur les positions 38-45 est délété, et chacun des autres Xaa est l'acide aminé se trouvant dans le GLP-1(7-37) natif.

13. Dérivé du GLP-1 selon la revendication 1, dans lequel Xaa sur les positions 26 et 34 est Arg, Xaa en position 36 est Lys, chacun des Xaa sur les positions 38-45 est délété, et chacun des autres Xaa est l'acide aminé se trouvant dans le GLP-1(7-37) natif.

14. Dérivé du GLP-1 selon la revendication 1, dans lequel Xaa en position 8 est Thr, Ser, Gly ou Val, Xaa en position 37 est Glu, Xaa en position 36 est Lys, chacun des Xaa sur les positions 38-45 est délété, et chacun des autres Xaa est l'acide aminé se trouvant dans le GLP-1(7-37) natif.

15. Dérivé du GLP-1 selon la revendication 1, dans lequel Xaa en position 18, 23 ou 27 est Lys, et Xaa sur les positions 26 et 34 est Arg, chacun des Xaa sur les positions 38-45 est délété, et chacun des autres Xaa est l'acide aminé se trouvant dans le GLP-1(7-37) natif.

16. Dérivé du GLP-1 selon la revendication 1, dans lequel Xaa en position 8 est Thr, Ser, Gly ou Val, Xaa en position 18, 23 ou 27 est Lys, et Xaa sur les positions 26 et 34 est Arg, chacun des Xaa sur les positions 38-45 est délété, et chacun des autres Xaa est l'acide aminé se trouvant dans le GLP-1(7-37) natif.

17. Dérivé du GLP-1 selon l'une quelconque des revendications 1 à 16, dans lequel sont présents trois substituants lipophiles.

18. Dérivé du GLP-1 selon l'une quelconque des revendications 1 à 16, dans lequel sont présents deux substituants lipophiles.

19. Dérivé du GLP-1 selon l'une quelconque des revendications 1 à 16, dans lequel un substituant lipophile est présent.

20. Dérivé du GLP-1 selon l'une quelconque des revendications 1 à 19, dans lequel un substituant lipophile est fixé au groupe amino du résidu d'acide aminé N-terminal du peptide GLP-1.

21. Dérivé du GLP-1 selon l'une quelconque des revendications 1 à 20, dans lequel un substituant lipophile est fixé au groupe carboxy du résidu d'acide aminé C-terminal du peptide GLP-1.

22. Dérivé du GLP-1 selon l'une quelconque des revendications 1 à 21, dans lequel un substituant lipophile est fixé au groupe carboxy qui fait partie du groupe R de l'Asp ou du Glu du peptide GLP-1.

23. Dérivé du GLP-1 selon l'une quelconque des revendications 1 à 22, dans lequel un substituant lipophile est fixé à un groupe ε-amino du Lys du peptide GLP-1.

24. Dérivé du GLP-1 selon l'une quelconque des revendications 1 à 24, dans lequel un substituant lipophile est fixé à un résidu d'acide aminé de telle sorte qu'un groupe carboxyle du substituant lipophile forme une liaison amide avec le groupe ε-amino du Lys du peptide GLP-1.

25. Dérivé du GLP-1 selon la revendication 24, dans lequel l'espaceur est un groupe acide (alcane à chaîne ramifiée)-α,ω-dicarboxylique ayant de 1 à 7 groupes méthylène, de préférence deux groupes méthylène, qui forme une liaison amide avec un groupe amino du peptide GLP-1 parent et une liaison amide avec un groupe amino du substituant lipophile.

26. Dérivé du GLP-1 selon la revendication 24, dans lequel l'espaceur est un résidu d'acide aminé à l'exception de Cys ou Met, ou un dipeptide tel que Gly-Lys.

27. Dérivé du GLP-1 selon la revendication 26, dans lequel le groupe ε-amino de Lys forme une liaison amide avec un groupe carboxylique du résidu acide aminé ou de l'espaceur du peptide, et un groupe amino du résidu d'acide aminé ou de l'espaceur dipeptide forme une liaison amide avec un groupe carboxyle du substituant lipophile.

28. Dérivé du GLP-1 selon l'une quelconque des revendications 24 à 27, dans lequel l'espaceur est le groupe γ-L-glutamyle, β-L-asparagyle, β-alanyle, glycyle ou α-(γ-aminobutanoyle).

29. Dérivé du GLP-1 selon l'une quelconque des revendications 1 à 28, dans lequel le substituant lipophile comprend un squelette cyclopentanophénanthrène partiellement ou entièrement hydrogéné.

30. Dérivé du GLP-1 selon les revendications 1 à 28, dans lequel le substituant lipophile est un groupe alkyle à chaîne droite ou ramifiée.

31. Dérivé du GLP-1 selon l'une quelconque des revendications 1 à 28, dans lequel le substituant lipophile est un groupe acyle d'un acide gras à chaîne droite ou ramifiée, de préférence un groupe acyle d'un acide gras à chaîne droite.

32. Dérivé du GLP-1 selon la revendication 32, dans lequel le groupe acyle est choisi parmi CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- et CH₃(CH₂)₂₂CO-.

33. Dérivé du GLP-1 selon la revendication 32, dans lequel le groupe acyle est le groupe tétradécanoyle.

34. Dérivé du GLP-1 selon la revendication 32, dans lequel le groupe acyle est le groupe hexadécanoyle.

35. Dérivé du GLP-1 selon la revendication 32, dans lequel le groupe acyle est le groupe octadécanoyle.

36. Dérivé du GLP-1 selon l'une quelconque des revendications précédentes, qui est choisi parmi
désamino-His⁷,Arg²⁶,Lys³⁴ (N^{ε}- (γ-glutamyl (N^{α}-hexadécanoyl)))GLP-1(7-37)-OH,
désamino-His⁷,Arg³⁴,Lys²⁶ (N^{ε}- (γ-glutamyl (N^{α}-hexadécanoyl)))GLP-1(7-37),
désamino-His⁷,Arg³⁴, Lys²⁶ (N^{ε}- (y-aminobutyroyl (N^{γ}-hexadécanoyl)))GLP-1(7-37),
désamino-His⁷,Arg³⁴,Lys²⁶ (N^{ε}- (β-alanyl (N^{γ}-hexadécanoyl)))GLP-1(7-37),
Arg³⁴Ala⁸ (N^{α}-(imidazol-4-ylprop-2-énoyl),
Lys²⁶(N^{ε}- (y-aminobutyrolyl (N^{γ}-hexadécanoyl))) GLP-1 (8-37),
Arg³⁴Ala⁸(N^{α}- (imidazol-4-ylacétyle),
Lys²⁶(N^{ε}- (y-aminobutyroyl (N^{γ}-hexadécanoyl))) GLP-1(8,37),
désamino-His⁷,Arg³⁴,Lys²⁶ (N^{ε}- (y-aminobutyroyl (N^{γ}-tétradécanoyl)))GLP-1(7-37), et
désamino-His⁷,Arg³⁴,Lys²⁶ (N^{ε}- (y-aminobutyroyl (N^{γ}-octadécanoyl)))GLP-1(7-37).

37. Dérivé du GLP-1 selon l'une quelconque des revendications 1 à 30, dans lequel le substituant lipophile est un groupe acyle d'un acide (alcane à chaîne droite ou ramifiée)-α,ω-dicarboxylique.

38. Dérivé du GLP-1 selon la revendication 37, dans lequel le groupe acyle est choisi dans le groupe comprenant HOOC(CH₂)₁₄CO-, HOOC(CH₂)₁₆CO-, HOOC(CH₂)₁₈CO-, HOOC(CH₂)₂₀CO- et HOOC(CH₂)₂₂CO-.

39. Dérivé du GLP-1 selon l'une quelconque des revendications 1 à 20, dans lequel le substituant lipophile avec l'espaceur fixé est un groupe de formule CH₃(CH₂)ₚNH-CO(CH₂)₂CO-, où p est un entier de 8 à 33, de préférence de 12 à 28.

40. Dérivé du GLP-1 selon l'une quelconque des revendications 1 à 20, dans lequel le substituant lipophile avec l'espaceur fixé est un groupe de formule CH₃(CH₂)ᵣCO-NHCH (COOH) (CH₂) ₂CO-, où r est un entier de 10 à 24.

41. Dérivé du GLP-1 selon l'une quelconque des revendications 1 à 20, dans lequel le substituant lipophile avec l'espaceur fixé est un groupe de formule CH₃ (CH₂)ₛCO-NHCH ((CH₂)₂COOH) CO-, dans laquelle s est un entier de 8 à 24.

42. Dérivé du GLP-1 selon l'une quelconque des revendications 1 à 20, dans lequel le substituant lipophile avec l'espaceur fixé est un groupe de formule -NHCH(COOH)(CH₂)₄NH-CO(CH₂)ᵤCH₃, où u est un entier de 8 à 18.

43. Dérivé du GLP-1 selon l'une quelconque des revendications 1 à 20, dans lequel le substituant lipophile avec l'espaceur fixé est un groupe de formule -NHCH (COOH) (CH₂)₄NH-COCH ((CH₂)₂COOH) NH-CO (CH₂)_{w}CH₃, où w est un entier de 10 à 16.

44. Dérivé du GLP-1 selon l'une quelconque des revendications 1 à 20, dans lequel le substituant lipophile avec l'espaceur fixé est un groupe de formule -NHCH (COOH) (CH₂)₄NH-CO(CH₂)₂CH(COOH)NH-CO(CH₂)ₓCH₃, où x est un entier de 10 à 16.

45. Dérivé du GLP-1 selon l'une quelconque des revendications 1 à 20, dans lequel le substituant lipophile avec l'espaceur fixé est un groupe de formule -NHCH(COOH)(CH₂)₄NH-CO(CH₂)₂CH(COOH)NH-CO(CH₂)_{y}CH₃, où y vaut zéro ou est un entier de 1 à 22.

46. Dérivé du GLP-1 selon l'une quelconque des revendications 1 à 45, qui présente une activité insulinotrope, une aptitude à diminuer le glucagon, une aptitude à supprimer la motilité gastrique, une aptitude à rétablir la compétence au glucose des cellules bêta, et/ou l'aptitude à supprimer l'appétit/réduire le poids.

47. Composition pharmaceutique comprenant un dérivé du GLP-1 selon l'une quelconque des revendications 1 à 46 et un véhicule ou excipient acceptable d'un point de vue pharmaceutique.

48. Composition pharmaceutique selon la revendication 47, qui comprend en outre un autre agent anti-diabétique.

49. Composition pharmaceutique selon la revendication 48, dans laquelle l'agent anti-diabétique est une insuline, de préférence l'insuline humaine.

50. Composition pharmaceutique selon la revendication 48, dans laquelle l'agent anti-diabétique est un agent hypoglycémiant.

51. Composition pharmaceutique selon la revendication 47, qui comprend en outre un autre agent anti-obésité.

52. Composition pharmaceutique selon la revendication 51, dans laquelle l'agent anti-obésité est choisi dans le groupe consistant en la leptine, l'amphétamine, la dexfenfluramine, la sibutramine, l'orlistate, les agonistes du CART, les antagonistes du NPY, les antagonistes de l'oréxine, les antagonistes H3, les agonistes du TNF, les agonistes du CRF, les antagonistes du CRF BP, les agonistes de l'urocortine, les agonistes β3, les agonistes du MSH, les agonistes du CCK, les inhibiteurs de la recapture de la sérotonine, les composés en mélange sérotonine et noradrénergiques, les agonistes 5HT, les agonistes de la bombésine, les antagonistes de la galanine, l'hormone de croissance, la somatostatine, le glucagon, les agonistes du TRH, les modulateurs de la protéine UCP2 ou UCP3, les agonistes de la leptine, les agonistes du DA (bromocriptine, doprexine), les inhibiteurs de la lipase/amylase, les modulateurs PPAR, les modulateurs PXR et les agonistes du TR β.

53. Utilisation d'un dérivé du GLP-1 selon l'une quelconque des revendications 1 à 46 pour la préparation d'un médicament ayant un profil d'action prolongé par rapport à celui du GLP-1(7-37), pour le traitement du diabète sucré non insulino-dépendant.

54. Utilisation d'un dérivé du GLP-1 selon l'une quelconque des revendications 1 à 46, pour la préparation d'un médicament ayant un profil d'action prolongé par rapport à celui du GLP-1(7-37), pour le traitement du diabète sucré insulino-dépendant.

55. Utilisation d'un dérivé du GLP-1 selon l'une quelconque des revendications 1 à 46, pour la préparation d'un médicament ayant un profil d'action prolongé par rapport à celui du GLP-1(737), pour le traitement de l'obésite.

56. Dérivé du GLP-1 selon l'une quelconque des revendications 1 à 46, pour utilisation en tant que médicament.

57. Dérivé du GLP-1 selon l'une quelconque des revendications 1 à 46 pour la préparation d'un médicament pour le traitement du diabète sucré insulino-dépendant ou non insulino-dépendant.

58. Dérivé du GLP-1 selon l'une quelconque des revendications 1 à 46 pour la préparation d'un médicament pour le traitement de l'obésité.

59. Dérivé du GLP-1 selon l'une quelconque des revendications 1 à 46 pour la préparation d'un médicament pour le traitement de la résistance à l'insuline.

60. Dérivé du GLP-1 selon l'une quelconque des revendications 1 à 46 pour utilisation dans le traitement du diabète sucré insulino-dépendant ou non insulino-dépendant.

61. Dérivé du GLP-1 selon l'une quelconque des revendications 1 à 46, pour utilisation dans le traitement de l'obésité.

62. Dérivé du GLP-1 selon l'une quelconque des revendications 1 à 46, pour utilisation dans le traitement de la résistance à l'insuline.
